# DEMANDE DE BREVET EUROPEEN

(11) **EP 4 733 385 A1**
(43) Date de publication de la demande: **29.04.2026**
(21) Numéro de dépôt: 25211286.7
(22) Date de dépôt: 27.10.2025
(51) Int. Cl.: C12N 1/12, C12P 7/6434, A23K 10/16, A23K 20/158, A23K 50/80, C12R 1/89

(54) **PRODUCTION DE BIOMASSE DE MICROORGANISMES**

(30) Priorité: 28.10.2024 FR 2411792
(71) Demandeur: Sofral Le Gouessant, 22400 Lamballe Armor (FR); Centre National de la Recherche Scientifique (CNRS), 75016 Paris (FR); Université de Bretagne Occidentale, 29200 Brest (FR); Institut de Recherche pour le Développement (IRD), 13002 Marseille 2 (FR); Institut Francais de Recherche pour l'Exploitation de la Mer (IFREMER), 29280 Plouzané (FR)
(72) Inventeur: MARCHAND, Yann, 22400 LAMBALLE (FR); SOUDANT, Philippe, 29200 BREST (FR); CHAUCHAT, Luc, 29200 BREST (FR)
(74) Mandataire: Regimbeau

(57) **Abrégé**

La présente invention concerne un procédé de préparation d'une biomasse de microorganismes du genre *Schizochytrium* ou *Aurantiochytrium* comprenant les étapes de :
a) ensemencement, dans un bioréacteur, d'un milieu de culture comprenant une saumure, de préférence une saumure 6% d'extrait sec, et de l'azote, de préférence à une concentration comprise entre 5 et 15 g/L de milieu de culture, avec un inoculum de microorganismes du genre *Schizochytrium* ou *Aurantiochytrium,* ladite saumure étant un coproduit du traitement de déminéralisation du petit-lait,
b) culture des microorganismes dans ledit milieu de culture dans des conditions, notamment de température, pH, agitation et/ou apport en oxygène, appropriées pour la croissance desdits microorganismes, de préférence en conditions non-axéniques, et
c) collecte de la biomasse obtenue à l'issue de l'étape b).

## Description

### DOMAINE DE L'INVENTION

La présente invention concerne la production de biomasse de microorganismes appartenant au genre *Schizochytrium* ou *Aurantiochytrium.*

### ETAT DE LA TECHNIQUE

Les acteurs de l'aquaculture ont besoin de structurer et maîtriser leurs filières d'approvisionnement de protéines (protéines végétales et farine de poisson), de lipides (huiles végétales et huiles de poissons), et autres matières premières destinées à nourrir les poissons, pour limiter leur dépendance aux importations. Ce besoin est d'autant plus marqué dans un secteur d'activité particulièrement concurrentiel où le coût des matières premières représente près de 80% du coût de production de l'aliment.

La raréfaction des ressources naturelles et en particulier halieutiques (Moomaw et al. (2017) Industrial Biotechnology 13 :243-243), ainsi que la demande croissante du marché à l'égard des Acides Gras Polyinsaturés (AGPI) n-3 ou oméga 3 à longues chaines (acide eicosapentaénoïque (EPA), acide docosahexaénoïque (DHA)), ont en effet engendré une augmentation notable de leur prix. Ainsi, la demande en Europe pour AGPI n-3 ou oméga 3, EPA et DHA, a atteint 1100 M€ en 2022 (contre 500 M€ en 2014).

Dans ce contexte, l'émergence d'une bioéconomie circulaire, permettant de produire de façon écoresponsable des matières premières naturelles, est dorénavant une approche privilégiée au sein des filières agricoles et agroalimentaires.

Les alternatives végétales (terrestres) aux protéines marines n'ont pas permis d'atteindre systématiquement des niveaux de performances de croissance des poissons équivalentes. De plus, leur utilisation est associée à une dégradation de certains paramètres métaboliques (cholestérolémie) et des activités protéolytiques (Tibaldi et al. (2006) Aquaculture 261 :182-193). En effet il a été démontré qu'à taux de protéines équivalent, l'incorporation de protéines végétales en remplacement des farines de poisson induit une baisse de performance significative (Mundheim et al. (2004) Aquaculture 236 :315-331). Cet impact négatif sur la croissance est lié à une mauvaise digestibilité des protéines (Opstvedt et al. (2003) Aquaculture 221 :365-379) causée par la présence de facteurs antinutritionnels tels les inhibiteurs de protéases, tannins, glucosinolates qui limitent l'utilisation en aquaculture de protéines d'origine végétale (Bu et al. (2018) Journal of the World Aquaculture Society 49:1068-1080).

L'utilisation des microalgues (ou de microorganismes anciennement catégorisés comme microalgues) comme source de DHA est apparue depuis plusieurs années comme une alternative aux huiles de poissons mais reste contrainte par les volumes et les prix de ce type de biomasse. Les résultats montrent que ces sources peuvent être envisagées pour remplacer partiellement ou totalement les AGPI n-3 à longue chaine issus des huiles de poissons des pêches minotières utilisées dans l'agroalimentaire (AFSSA Saisine n° 2008-SA-0316 (2008)).

Or, la culture hétérotrophe de microorganismes appartenant au genre *Schizochytrium* ou *Aurantiochytrium* (anciennement considérés comme microalgues et autorisés à être commercialisés sous cette ancienne dénomination) présentent des difficultés techniques inhérentes à cette culture, les facteurs d'échecs résidant essentiellement dans le dimensionnement et le seuil critique à atteindre pour que l'entité industrielle puisse être rentable et couvrir la forte demande en ingrédients durables et soutenables pour l'aquaculture.

Il existe donc toujours un besoin important d'alternatives aux huiles de poisson, qui puissent être produites de manière écoresponsable tout en étant rentable pour les industriels.

La présente invention répond à ce besoin.

### EXPOSE DE L'INVENTION

La présente invention résulte de la découverte surprenante par les inventeurs qu'il était possible de produire de manière efficace et écoresponsable, une biomasse de microorganismes appartenant au genre *Schizochytrium* ou *Aurantiochytrium,* en utilisant un coproduit de l'industrie laitière comme substrat.

La biomasse ainsi obtenue présente un profil lipidique particulièrement adapté pour l'alimentation du bétail ainsi que l'alimentation aquacole, car riche en lipides polaires et en forme phospholipide d'omega 3. Elle permet donc de remplacer efficacement les ingrédients issus de la pêche minotière dans cette alimentation, ce qui est un avantage important d'un point de vue environnement et économique.

Le procédé développé par les inventeurs a de plus l'avantage de pouvoir être mis en œuvre dans des conditions non-axéniques, ce qui permet de réduire des surcoûts de fonctionnement tels que ceux liés à des obligations de stérilisation.

De plus, l'utilisation, dans ce procédé de production d'un coproduit de l'industrie laitière, sans valeur ajoutée et dont la seule destination finale est la station d'épuration, le rend particulièrement intéressant d'un point de vue économique et environnemental.

Enfin, ce procédé permet de produire la biomasse d'intérêt dans un délai court, ce qui est également avantageux d'un point de vue économique.

La présente invention concerne ainsi un procédé de préparation d'une biomasse de microorganismes du genre *Schizochytrium* ou *Aurantiochytrium* comprenant les étapes de :
a) ensemencement, dans un bioréacteur, d'un milieu de culture comprenant une saumure, de préférence une saumure 6% d'extrait sec, et de l'azote, de préférence à une concentration comprise entre 5 et 15 g/L de milieu de culture, avec un inoculum de microorganismes du genre *Schizochytrium* ou *Aurantiochytrium,* ladite saumure étant un coproduit du traitement de déminéralisation du petit-lait,
b) culture des microorganismes dans ledit milieu de culture dans des conditions, notamment de température, pH, agitation et/ou apport en oxygène, appropriées pour la croissance desdits microorganismes, de préférence en conditions non-axéniques, et
c) collecte de la biomasse obtenue à l'issue de l'étape b).

Un autre objet de l'invention concerne également une biomasse de microorganismes du genre *Schizochytrium* ou *Aurantiochytrium* susceptible d'être obtenue par le procédé de préparation de biomasse selon l'invention.

La présente invention concerne également une biomasse de microorganismes du genre *Schizochytrium* ou *Aurantiochytrium* caractérisée en ce qu'elle présente le profil lipidique suivant :
- entre 55 et 65% d'acides gras totaux sous forme de lipides polaires, et
- au moins une des caractéristiques suivantes :
   - entre 18 et 25 µg d'acides gras totaux / mg de poids de sec de biomasse,
   - entre 35 et 40% d'acide docosahexaénoïque (DHA) dans les lipides neutres,
   - un ratio DHA / acide docosapentaénoïque (DPA) dans les lipides neutres compris entre 4 et 4,5,
   - entre 45 et 55% de DHA dans les lipides polaires, et
   - un ratio DHA / DPA dans les lipides polaires compris entre 3 et 4.

Un autre objet de l'invention concerne l'utilisation de la biomasse de microorganismes du genre *Schizochytrium* ou *Aurantiochytrium* selon l'invention comme ingrédient dans un aliment pour animaux d'élevage, notamment un aliment aquacole, en particulier dans un aliment pour bar d'élevage.

La présente invention concerne également un aliment pour animaux d'élevage, notamment aquacole, en particulier pour bar d'élevage, comprenant entre 15% et 30% en poids de biomasse de microorganismes du genre *Schizochytrium* ou *Aurantiochytrium* selon l'invention.

Un autre objet de l'invention concerne une méthode d'élevage de poissons, en particulier de bars, plus particulièrement de juvéniles de bars, comprenant la fourniture, notamment pendant 5 à 10 semaines, de préférence tous les jours, de la biomasse de microorganismes du genre *Schizochytrium* ou *Aurantiochytrium* selon l'invention auxdits poissons, de préférence sous la forme de l'aliment selon l'invention, de préférence à une dose moyenne journalière de 2% à 3% du poids vif.

### DESCRIPTION DES FIGURES

La Figure 1 représente les cinétiques de la matière sèche (MS) et de la concentration cellulaire (Cell/ml) (Moyenne et écart type) obtenues à l'exemple **1**
La Figure 2 représente les pourcentages d'EPA (20 :5n-3) et de DHA (22 :6n-3) dans les lipides neutres des muscles de juvéniles nourris avec un aliment témoin (« Control »), et un aliment contenant 15% de biomasse selon l'invention (« Microalgae »).
La Figure 3 représente les pourcentages d'EPA (20 :5n-3) et de DHA (22 :6n-3) dans les lipides polaires des muscles de juvéniles nourris avec un aliment témoin (« Control »), et un aliment contenant 15% de biomasse selon l'invention (« Microalgae »).
La Figure 4 représente les cinétiques des poids moyens des juvéniles de bar (g/poisson) par modalité au cours de l'essai de l'exemple 3.

### DESCRIPTION DETAILLEE DE L'INVENTION

### Microorganismes du genre Schizochytrium ou Aurantiochytrium

La présente invention met en œuvre des microorganismes du genre *Schizochytrium* ou *Aurantiochytrium.*

Par « **microorganisme du genre** ***Schizochytrium** »,* on entend ici un genre d'eucaryotes unicellulaires de la famille des *Thraustochytriaceae,* que l'on trouve dans les habitats marins côtiers.

Les microorganismes du genre *Schizochytrium* sont bien connus de l'homme du métier et incluent les microorganismes des espèces *Schizochytrium aggregatum, Schizochytrium limacinum,* et *Schizochytrium minutum.*

Par « **microorganisme du genre** ***Aurantiochytrium** »,* on entend ici un genre d'eucaryotes unicellulaires de la famille des *Thraustochytriaceae.*

Les microorganismes du genre *Aurantiochytrium* sont bien connus de l'homme du métier et incluent notamment les microorganismes des espèces *Aurantiochytrium mangrovei* (anciennement *Schizochytrium mangrovei*), *Aurantiochytrium limacinum* et *Aurantiochytrium sp..*

Dans un mode de réalisation particulier, les microorganismes du genre *Schizochytrium* ou *Aurantiochytrium* sont des microorganismes de l'espèce *Aurantiochytrium mangrovei.*

Dans un mode de réalisation particulier, le microorganisme de l'espèce *Aurantiochytrium mangrovei* est la souche d'A. *mangrovei* RCC893 (Roscoff Culture Collection, France).

Comme il est bien connu de l'homme du métier, les microorganismes du genre *Schizochytrium* ou *Aurantiochytrium* étaient anciennement considérés comme des microalgues et autorisés à être commercialisés sous cette ancienne dénomination.

Par « **biomasse** », on entend ici une masse, quantité, ou volume, comprenant des microorganismes vivants.

### Procédé de préparation de biomasse

La présente invention concerne un procédé de préparation d'une biomasse de microorganismes du genre *Schizochytrium* ou *Aurantiochytrium,* tels que définis dans la section *« Microorganismes du genre Schizochytrium ou Aurantiochytrium »* ci-dessus, comprenant les étapes de :
a) ensemencement, dans un bioréacteur, d'un milieu de culture comprenant une saumure et de l'azote avec un inoculum de microorganismes du genre *Schizochytrium* ou *Aurantiochytrium,* ladite saumure étant un coproduit du traitement de déminéralisation du petit-lait,
b) culture des microorganismes dans ledit milieu de culture dans des conditions appropriées pour la croissance desdits microorganismes, et
c) collecte de la biomasse obtenue à l'issue de l'étape b).

### Etape d'ensemencement

L'étape a) du procédé selon l'invention consiste à ensemencer, dans un bioréacteur un milieu de culture comprenant une saumure et de l'azote avec un inoculum de microorganismes du genre *Schizochytrium* ou *Aurantiochytrium,* tels que définis dans la section *« Microorganismes du genre Schizochytrium ou Aurantiochytrium »* ci-dessus.

Par « **ensemencer** » ou « **ensemencement** », on entend ici l'introduction de microorganismes dans un milieu de culture, qui se multiplieront une fois implantés dans ce milieu de culture.

Par « **inoculum** », on entend ici la matière utilisée pour l'ensemencement, par exemple une composition comprenant les microorganismes, qui sont utilisés pour amorcer un procédé d'intérêt. Ainsi « **ensemencer** » signifie transférer l'inoculum dans le milieu utilisé dans le procédé d'intérêt.

L'inoculum peut être sous forme liquide, sous forme sèche ou sous forme essentiellement sèche. Dans un mode de réalisation préféré, l'inoculum est sous forme liquide.

La quantité de microorganismes dans l'inoculum pourra être déterminée par l'homme du métier selon la taille du bioréacteur à ensemencer, la nature du milieu de culture, les conditions de culture, par exemple.

Ainsi, par exemple, dans un bioréacteur de 800 L, on peut utiliser un inoculum de 2 x 8 L de culture de microorganismes du genre *Schizochytrium* ou *Aurantiochytrium,* obtenu par exemple après 24 h de culture dans un milieu YEP.

Par « **bioréacteur** », on entend ici un dispositif ou système qui supporte la croissance de cellules dans un milieu de culture. En particulier, le bioréacteur peut être un récipient dans lequel un procédé biologique est mis en oeuvre, qui implique des organismes ou microorganismes. Ce procédé peut être aérobie ou anaérobie.

Le bioréacteur peut être de toute forme appropriée. Ainsi, de préférence, le bioréacteur utilisé dans le cadre de l'invention est de forme cylindrique.

Le bioréacteur peut être d'une taille pouvant aller de quelques litres à plusieurs mètres cubes. De préférence, le bioréacteur utilisé dans le cadre de l'invention a un volume de 500 L à 1000 L, de préférence un volume de 800 L.

Le bioréacteur peut être en acier inoxydable ou en tout autre matériau adapté à la culture d'organismes ou de microorganismes. De préférence, le bioréacteur utilisé dans le cadre de l'invention est en poly-méthyl-méthacrylate (PMMA).

Selon le mode de fonctionnement choisi, le bioréacteur peut être classé comme un bioréacteur pour culture en batch, en fed-batch ou continu. De préférence, le bioréacteur utilisé dans le cadre de l'invention est conçu pour une culture en batch, de préférence une culture en batch de 400 L à 800 L, de préférence une culture en batch de 500 L.

Le bioréacteur peut être équipé d'une ou plusieurs entrées, pour la fourniture de nouveau milieu frais aux cellules par exemple, et d'une ou plusieurs sorties pour récupérer le produit ou vider le bioréacteur.

Le bioréacteur peut en outre être équipé de dispositifs permettant de surveiller, contrôler et/ou réguler les conditions expérimentales telles que le gaz (air, oxygène, azote, dioxyde de carbone), les débits, la température, le pH, le niveau d'exposition à la lumière, les niveaux d'oxygène dissous, la vitesse d'agitation et le taux de circulation.

Ainsi, de préférence le bioréacteur utilisé dans le cadre de l'invention comprend un dispositif permettant de surveiller, contrôler et/ou réguler la température du milieu de culture. Un exemple de dispositif permettant de surveiller, contrôler et/ou réguler la température du milieu de culture est un chauffage électrique, de préférence en acier inoxydable, pouvant être immergé dans le milieu de culture.

De préférence, le bioréacteur utilisé dans le cadre de l'invention comprend un dispositif permettant de surveiller, contrôler et/ou réguler le pH du milieu de culture. Un exemple de dispositif permettant de surveiller, contrôler et/ou réguler le pH du milieu de culture est une sonde pH.

De préférence, le bioréacteur utilisé dans le cadre de l'invention comprend un dispositif permettant l'apport d'eau pendant l'étape de culture et/ou pour nettoyer le bioréacteur. Un exemple de dispositif permettant l'apport d'eau est une pompe, éventuellement associée à une buse rotative.

De préférence, le bioréacteur utilisé dans le cadre de l'invention comprend un dispositif permettant d'agiter le milieu de culture et/ou d'apporter de l'oxygène dans le milieu de culture. Un exemple de dispositif permettant d'agiter le milieu de culture et/ou d'apporter de l'oxygène dans le milieu de culture est un bulleur, de préférence situé au fond du bioréacteur, et formant un flux d'air, de préférence filtré, par exemple à 0,2 µm, et/ou un débitmètre massique permettant de contrôler le débit d'air.

Par « **milieu de culture** », on entend ici tout milieu capable de supporter la croissance, le maintien, la propagation et/ou l'expansion de cellules dans un environnement artificiel in *vitro.* Les milieux de culture peuvent être optimisés pour une utilisation spécifique de la culture cellulaire, par exemple pour promouvoir la production de biomasse ou pour promouvoir la production de composés d'intérêt.

Un milieu de culture apporte typiquement au moins un des composants suivants aux cellules cultivées : une source d'énergie (généralement sous la forme d'un sucre tel que du glucose), un ou plusieurs acides aminés essentiels, des vitamines et/ou d'autres composés organiques généralement nécessaires à de faibles concentrations, des lipides ou des acides gras libres ; et des éléments traces.

De préférence, le milieu de culture utilisé dans le cadre de l'invention est un milieu de culture liquide.

Le milieu de culture utilisé dans le cadre de l'invention comprend une saumure et de l'azote.

Par « **saumure** », on entend ici une solution aqueuse saturée de sel. Le sel est de préférence est un sel minéral.

La saumure utilisée dans le cadre de l'invention est de préférence une saumure à 3 à 10% d'extrait sec, de préférence de 4 à 9% d'extrait sec, de préférence de 5 à 8% d'extrait sec, ou de 6 à 7% d'extrait sec. De préférence entre toutes, la saumure utilisée dans le cadre de l'invention est une saumure à 6% d'extrait sec.

Par « **extrait sec** », on entend ici le pourcentage de résidu déshydraté de la composition après séchage, en masse par rapport au poids de composition initiale.

La saumure utilisée dans le cadre de l'invention est un coproduit du traitement de déminéralisation du petit-lait. L'utilisation d'un tel coproduit est particulièrement avantageux sur le plan environnemental et économique puisqu'elle permet de valoriser un coproduit qui était sans valeur ajoutée et dont la seule finalité était la station d'épuration, mais dont l'utilisation pour la production d'ingrédients destinés à l'alimentation animale est autorisée.

Par « **petit-lait** » ou « **lactosérum** », on entend ici la partie liquide issue du processus de coagulation du lait. Le petit-lait est composé typiquement d'environ 94% d'eau, de 4 à 5 % de lactose, de protéines solubles ainsi que de sels minéraux et de vitamines liposolubles et hydrosolubles.

Par « **traitement de déminéralisation du petit-lait** », on entend ici le procédé consistant à retirer les sels minéraux du petit-lait, pour produire du petit-lait (ou lactosérum) déminéralisé. Les techniques de déminéralisation du petit-lait sont bien connues de l'homme du métier et comprennent l'ultrafiltration, l'osmose inverse, l'électrodialyse, l'échange d'ions ou une combinaison de ces techniques.

Par « **coproduit du traitement de déminéralisation du petit-lait** », on entend ici la matière créée de façon inévitable par le processus de fabrication du petit-lait (ou lactosérum) déminéralisé à partir de petit-lait par déminéralisation.

Le coproduit du traitement de déminéralisation du petit-lait est ainsi typiquement majoritairement constitué de sels minéraux, en particulier de potassium, de sodium, de calcium et de magnésium.

La saumure utilisée dans le cadre de l'invention est particulièrement intéressante car elle est riche en sels et en phosphore. Or, le phosphore est un élément dont la quantité n'est pas illimitée à l'échelle mondiale. L'apport de phosphore via un coproduit qui était sans valeur ajoutée et dont la seule destination finale était la station d'épuration est donc particulièrement avantageux.

Ainsi la saumure utilisée dans le cadre de l'invention comprend de préférence du potassium (en particulier entre 15 g et 25 g pour 100 g d'extrait sec, plus particulièrement entre 20 g et 21 g pour 100 g d'extrait sec), du sodium (en particulier entre 3 g et 7 g pour 100 g d'extrait sec, plus particulièrement entre 5 g et 6 g pour 100 g d'extrait sec), du calcium (en particulier entre 1 g et 4 g pour 100 g d'extrait sec, plus particulièrement entre 2 g et 3 g pour 100 g d'extrait sec), du magnésium (en particulier entre 300 mg et 600 mg pour 100 g d'extrait sec, plus particulièrement entre 400 mg et 500 mg pour 100 g d'extrait sec), et du phosphore (en particulier entre 500 mg et 3 g pour 100 g d'extrait sec, plus particulièrement entre 1 g et 2 g pour 100 g d'extrait sec).

De plus, la saumure utilisée dans le cadre de l'invention apporte également l'eau au milieu de culture. L'apport d'une eau venant d'un coproduit sans valeur ajoutée et dont la seule finalité était la station d'épuration est donc également particulièrement vertueux en termes de gestion environnementale de l'eau.

L'azote utilisé dans le cadre de l'invention peut être apporté par n'importe quelle source appropriée, bien connue de l'homme du métier. Des exemples de sources d'azote comprennent l'azote organique, l'azote ammoniacal, l'azote nitreux et l'azote nitrique.

De préférence, l'azote utilisé dans le cadre de l'invention est de l'azote ammoniacal.

Par « **azote ammoniacal** », on entend ici l'azote présent sous forme d'ions ammonium NH₄⁺. L'azote ammoniacal peut en particulier être apporté sous forme de sels d'ammonium, tels que le sulfate d'ammonium (NH₄)₂SO₄, le nitrate d'ammonium NH₄NO₃, ou le chlorure d'ammonium NH₄Cl. De préférence, l'azote ammoniacal utilisé dans le cadre de l'invention est sous forme de sulfate d'ammonium.

De préférence, l'azote, en particulier l'azote ammoniacal, plus particulièrement l'azote ammoniacal sous forme de sulfate d'ammonium, est présent dans le milieu de culture à une concentration comprise entre 5 et 15 g/L de milieu de culture, plus particulièrement entre 6 et 14 g/L, entre 7 et 13 g/L, entre 8 et 12 g/L, ou de préférence encore entre 9 et 11 g/L de milieu de culture. De manière particulièrement préférée, l'azote, en particulier l'azote ammoniacal, plus particulièrement l'azote ammoniacal sous forme de sulfate d'ammonium, est présent dans le milieu de culture à une concentration comprise entre 10 et 11 g/L de milieu de culture.

De préférence, l'azote, en particulier l'azote ammoniacal, plus particulièrement l'azote ammoniacal sous forme de sulfate d'ammonium, utilisé dans le cadre de l'invention est apporté dans le milieu de culture préalablement mélangé dans la saumure telle que définie ci-dessus.

Typiquement, l'azote, en particulier l'azote ammoniacal, plus particulièrement l'azote ammoniacal sous forme de sulfate d'ammonium, utilisé dans le cadre de l'invention est mélangé dans la saumure, puis solubilisé dans la saumure, par exemple au moins d'une pompe centrifuge. L'azote, en particulier l'azote ammoniacal, plus particulièrement l'azote ammoniacal sous forme de sulfate d'ammonium, solubilisé dans la saumure peut ensuite être filtré avant d'être transféré dans le bioréacteur.

Dans un mode de réalisation particulier, le milieu de culture comprend en outre au moins un substrat carboné et/ou des métaux traces et/ou au moins une vitamine.

Dans un mode de réalisation particulier, le milieu de culture comprend en outre au moins un substrat carboné et des métaux traces.

Dans un mode de réalisation particulier, le milieu de culture comprend en outre au moins un substrat carboné, des métaux traces et au moins une vitamine.

Par « **substrat carboné** », on entend ici une source de carbone qui peut être métabolisée par les microorganismes de la présente invention. Des exemples de substrat carboné incluent des monosaccharides, des oligosaccharides, des polysaccharides, et des mélanges de ceux-ci. Plus particulièrement, des substrats carbonés incluent par exemple des substrats comprenant du glucose, du fructose, du galactose, du mannose, du mannitol, du saccharose, de l'amidon, des hydrolysats d'amidon et des molasses.

Dans un mode de réalisation particulier, le substrat carboné utilisé dans le cadre de l'invention est un sirop de glucose. Dans un mode de réalisation plus particulier, le substrat carboné utilisé dans le cadre de l'invention est un sirop de glucose comprenant du fructose. Dans un mode de réalisation particulier, le substrat carboné utilisé dans le cadre de l'invention est un sirop de glucose comprenant de 25% à 30% de fructose. Dans un mode de réalisation particulier, le substrat carboné utilisé dans le cadre de l'invention est un sirop de glucose comprenant du fructose et du dextrose et/ou du maltose. Dans un mode de réalisation particulier, le substrat carboné utilisé dans le cadre de l'invention est un sirop de glucose comprenant de 25% à 30% de fructose, de 40% à 45% de maltose et de 17% à 33% de maltose. Dans un mode de réalisation particulier, le substrat carboné utilisé dans le cadre de l'invention présente un équivalent dextrose comprise entre 81,0 et 86,0.

Des exemples de substrats carbonés appropriés pouvant être utilisés dans le cadre de l'invention incluent l'Isosweet 470 commercialisé par Tereos (France).

De préférence, le milieu de culture utilisé dans le cadre de l'invention comprend entre 20 g/L et 50 g/L de substrat carboné, en particulier de sirop de glucose, plus particulièrement de sirop de glucose comprenant du fructose, de manière d'avantage préférée entre 45 g/L et 50 g/L de substrat carboné, en particulier de sirop de glucose, plus particulièrement de sirop de glucose comprenant du fructose, de manière plus préférée entre 48 g/L et 49 g/L de substrat carboné, en particulier de sirop de glucose, plus particulièrement de sirop de glucose comprenant du fructose.

Par « **métaux traces** » ou « **éléments traces métalliques** » on entend ici des sels inorganiques métalliques, à l'exception de NaCl, qui sont présents des quantités traces dans le système, mais peuvent être essentiels à la croissance et la survie des microorganismes. Des exemples de métaux traces incluent le manganèse, le zinc, le fer, le cuivre, le cobalt, le bore, le molybdène, le sélénium et le nickel.

Ainsi, dans un mode de réalisation préféré, le milieu de culture comprend en outre des métaux traces choisis parmi le manganèse, le zinc, le fer, le cuivre, le cobalt, le bore, le molybdène, le sélénium, le nickel, et leurs combinaisons. Dans un mode de réalisation particulièrement préféré, le milieu de culture comprend en outre du manganèse, du zinc, du fer, du cuivre, du cobalt, du bore, du molybdène, du sélénium et du nickel.

Le cobalt peut typiquement être apporté sous forme CoCl₂, 6H₂O. De préférence, le cobalt, en particulier sous forme CoCl₂, 6H₂O, est présent dans le milieu de culture à une concentration comprise entre 0,01 mg/L et 0,03 mg/L, de manière préférée entre 0,02 mg/L et 0,025 mg/L.

Le cuivre peut typiquement être apporté sous forme CuSO,H₂O. De préférence, le cuivre, en particulier sous forme CuSO,H₂O, est présent dans le milieu de culture à une concentration comprise entre 0,4 mg/L et 80 mg/L, de manière préférée entre 75 mg/L et 76 mg/L.

Le bore peut typiquement être apporté sous forme H₃BO₃. De préférence, le bore, en particulier sous forme H₃BO₃, est présent dans le milieu de culture à une concentration comprise entre 0 mg/L et 10 mg/L, de manière préférée entre 9 mg/L et 10 mg/L.

Le manganèse peut typiquement être apporté sous forme MnCl₂, 4H₂O. De préférence, le manganèse, en particulier sous forme MnCl₂, 4H₂O, est présent dans le milieu de culture à une concentration comprise entre 2 mg/L et 10 mg/L, de manière préférée entre 4 mg/L et 5 mg/L.

Le molybdène peut typiquement être apporté sous forme Na₂MoO₄, 2H₂O. De préférence, le molybdène, en particulier sous forme Na₂MoO₄, 2H₂O, est présent dans le milieu de culture à une concentration comprise entre 0 mg/L et 0,07 mg/L, de manière préférée entre 0,05 mg/L et 0,06 mg/L.

Le sélénium peut typiquement être apporté sous forme Na₂SeO₃. De préférence, le sélénium, en particulier sous forme Na₂SeO₃, est présent dans le milieu de culture à une concentration comprise entre 0,01 mg/L et 0,06 mg/L, de manière préférée entre 0,02 mg/L et 0,03 mg/L.

Le nickel peut typiquement être apporté sous forme NiSO₄, 6H₂O. De préférence, le nickel, en particulier sous forme NiSO₄, 6H₂O, est présent dans le milieu de culture à une concentration comprise entre 0 mg/L et 0,02 mg/L, de manière préférée entre 0,01 mg/L et 0,02 mg/L.

Le zinc peut typiquement être apporté sous forme ZnSO₄, 7H₂O. De préférence, le zinc, en particulier sous forme ZnSO₄, 7H₂O, est présent dans le milieu de culture à une concentration comprise entre 0,4 mg/L et 70 mg/L, de manière préférée entre 65 mg/L et 67 mg/L.

Le fer peut typiquement être apporté sous forme FeSO₄, 7H₂O. De préférence, le fer, en particulier sous forme FeSO₄, 7H₂O, est présent dans le milieu de culture à une concentration comprise entre 20 mg/L et 60 mg/L, de manière préférée entre 40 mg/L et 45 mg/L.

Dans un mode de réalisation particulièrement préféré, le milieu de culture comprend en outre :
- du cobalt, de préférence sous forme CoCl₂, 6H₂O, à une concentration comprise entre 0,01 mg/L et 0,03 mg/L, en particulier entre 0,02 mg/L et 0,025 mg/L ;
- du cuivre, de préférence sous forme CuSO,H₂O, à une concentration comprise entre 0,4 mg/L et 80 mg/L, en particulier entre 75 mg/L et 76 mg/L ;
- du bore, de préférence sous forme H₃BO₃, à une concentration comprise entre 0 mg/L et 10 mg/L, en particulier entre 9 mg/L et 10 mg/L ;
- du manganèse, de préférence sous forme MnCl₂, 4H₂O, à une concentration comprise entre 2 mg/L et 10 mg/L, en particulier entre 4 mg/L et 5 mg/L ;
- du molybdène, de préférence sous forme Na₂MoO₄, 2H₂O, à une concentration comprise entre 0 mg/L et 0,07 mg/L, en particulier entre 0,05 mg/L et 0,06 mg/L ;
- du sélénium, de préférence sous forme Na₂SeO₃, à une concentration comprise entre 0,01 mg/L et 0,06 mg/L, en particulier entre 0,02 mg/L et 0,03 mg/L ;
- du nickel, de préférence sous forme NiSO₄, 6H₂O, à une concentration comprise entre 0 mg/L et 0,02 mg/L, en particulier entre 0,01 mg/L et 0,02 mg/L ;
- du zinc, de préférence sous forme ZnSO₄, 7H₂O, à une concentration comprise entre 0,4 mg/L et 70 mg/L, en particulier entre 65 mg/L et 67 mg/L ; et
- du fer, de préférence sous forme FeSO₄, 7H₂O, à une concentration comprise entre 20 mg/L et 60 mg/L, en particulier entre 40 mg/L et 45 mg/L.

Par « **vitamine** », on entend ici des composés organiques traces qui sont essentiels à la croissance normale et à la nutrition des organismes vivants. Les vitamines étant classées selon leur activité biologique et non selon leur structure, chaque « vitamine » est un descripteur générique et fait référence à un certain nombre de composés qui présentent tous l'activité biologique associée à une vitamine particulière.

Des exemples de vitamine incluent en particulier la vitamine A (rétinol, rétinal, ou acide rétinoïque), la vitamine B1 (thiamine), la vitamine B2 (riboflavine), la vitamine B3 (acide nicotinique, aussi appelée niacine), la vitamine B5 (acide pantothénique), la vitamine B6 (pyridoxine), la vitamine B8 (biotine), la vitamine B9 (acide folique ou folates), la vitamine B12 (cobalamine), la vitamine C (acide ascorbique), la vitamine D (incluant ergocalciférol et cholécalciférol), la vitamine E (incluant les tocophérols et les tocotriénols) et la vitamine K (incluant la phytoménadione et les ménaquinones).

Ainsi, dans un mode de réalisation préféré, le milieu de culture comprend en outre au moins une vitamine choisie dans le groupe constitué de la vitamine A, la vitamine B1, la vitamine B2, la vitamine B3, la vitamine B5, la vitamine B6, la vitamine B8, la vitamine B9, la vitamine B12, la vitamine C, la vitamine D, la vitamine E et la vitamine K, de préférence choisie dans le groupe constitué de la vitamine B1, la vitamine B3, la vitamine B5, la vitamine B6, la vitamine B8, la vitamine B9, la vitamine B12 et la vitamine C.

Dans un mode de réalisation préféré, le milieu de culture comprend en outre de la vitamine B1, de la vitamine B3, de la vitamine B5, de la vitamine B6, de la vitamine B8, de la vitamine B9, de la vitamine B12 et de la vitamine C.

De préférence la vitamine B1 est présente dans le milieu de culture à une concentration comprise entre 0,005 mg/L et 0,5 mg/L, en particulier entre 0,4 mg/L et 0,45 mg/L.

De préférence, la vitamine B3 est présente dans le milieu de culture à une concentration comprise entre 0,04 mg/L et 25 mg/L, en particulier entre 20 mg/L et 24 mg/L.

De préférence, la vitamine B5 est présente dans le milieu de culture à une concentration comprise entre 0 mg/L et 10 mg/L, en particulier entre 8 mg/L et 9 mg/L.

De préférence, la vitamine B6 est présente dans le milieu de culture à une concentration comprise entre 0 mg/L et 4 mg/L, en particulier entre 3 mg/L et 4 mg/L.

De préférence, la vitamine B8 est présente dans le milieu de culture à une concentration comprise entre 0 mg/L et 0,05 mg/L, en particulier entre 0,04 mg/L et 0,045 mg/L.

De préférence, la vitamine B9 est présente dans le milieu de culture à une concentration comprise entre 0,008 mg/L et 1 mg/L, en particulier entre 0,8 mg/L et 0,9 mg/L.

De préférence, la vitamine B12 est présente dans le milieu de culture à une concentration comprise entre 0,0007 mg/L et 0,03 mg/L, en particulier entre 0,02 mg/L et 0,03 mg/L.

De préférence, la vitamine C est présente dans le milieu de culture à une concentration comprise entre 0,3 mg/L et 160 mg/L, en particulier entre 145 mg/L et 155 mg/L.

Dans un mode de réalisation particulièrement préféré, le milieu de culture comprend en outre :
- de la vitamine B1 à une concentration comprise entre 0,005 mg/L et 0,5 mg/L, en particulier entre 0,4 mg/L et 0,45 mg/L;
- de la vitamine B3 à une concentration comprise entre 0,04 mg/L et 25 mg/L, en particulier entre 20 mg/L et 24 mg/L;
- de la vitamine B5 à une concentration comprise entre 0 mg/L et 10 mg/L, en particulier entre 8 mg/L et 9 mg/L ;
- de la vitamine B6 à une concentration comprise entre 0 mg/L et 4 mg/L, en particulier entre 3 mg/L et 4 mg/L ;
- de la vitamine B8 à une concentration comprise entre 0 mg/L et 0,05 mg/L, en particulier entre 0,04 mg/L et 0,045 mg/L ;
- de la vitamine B9 à une concentration comprise entre 0,008 mg/L et 1 mg/L, en particulier entre 0,8 mg/L et 0,9 mg/L ;
- de la vitamine B12 à une concentration comprise entre 0,0007 mg/L et 0,03 mg/L, en particulier entre 0,02 mg/L et 0,03 mg/L ; et
- de la vitamine C à une concentration comprise entre 0,3 mg/L et 160 mg/L, en particulier entre 145 mg/L et 155 mg/L.

Dans un mode de réalisation particulièrement préféré, le milieu de culture utilisé dans le cadre de l'invention comprend :
- entre 10 g/L et 11 g/L de milieu de culture d'azote, en particulier d'azote ammoniacal, plus particulièrement d'azote ammoniacal sous forme de sulfate d'ammonium, solubilisé dans de la saumure, en particulier une saumure à 6% d'extrait sec, qui est un coproduit du traitement de déminéralisation du petit-lait,
- entre 48 g/L et 49 g/L de substrat carboné, en particulier de sirop de glucose, plus particulièrement de sirop de glucose comprenant du fructose,
- entre 0,02 mg/L et 0,025 mg/L de cobalt, de préférence sous forme CoCl₂, 6H₂O,
- entre 75 mg/L et 76 mg/L de cuivre, de préférence sous forme CuSO,H₂O,
- entre 9 mg/L et 10 mg/L de bore, de préférence sous forme H₃BO₃,
- entre 4 mg/L et 5 mg/L de manganèse, de préférence sous forme MnCl₂, 4H₂O,
- entre 0,05 mg/L et 0,06 mg/L de molybdène, de préférence sous forme Na₂MoO₄, 2H₂O,
- entre 0,02 mg/L et 0,03 mg/L de sélénium, de préférence sous forme Na₂SeO₃,
- entre 0,01 mg/L et 0,02 mg/L de nickel, de préférence sous forme NiSO₄, 6H₂O,
- entre 65 mg/L et 67 mg/L de zinc, de préférence sous forme ZnSO₄, 7H₂O,
- entre 40 mg/L et 45 mg/L de fer, de préférence sous forme FeSO₄, 7H₂O,
- entre 0,4 mg/L et 0,45 mg/L de vitamine B1,
- entre 20 mg/L et 24 mg/L de vitamine B3,
- entre 8 mg/L et 9 mg/L de vitamine B5,
- entre 3 mg/L et 4 mg/L de vitamine B6,
- entre 0,04 mg/L et 0,045 mg/L de vitamine B8,
- entre 0,8 mg/L et 0,9 mg/L de vitamine B9,
- entre 0,02 mg/L et 0,03 mg/L de vitamine B12, et
- entre 145 mg/L et 155 mg/L de vitamine C.

### Etape de culture

L'étape b) du procédé selon l'invention consiste à cultiver les microorganismes du genre *Schizochytrium* ou *Aurantiochytrium* définis dans la section « *Microorganismes du genre Schizochytrium ou Aurantiochytrium »* ci-dessus, dans le milieu de culture, défini dans la section « Etape d'ensemencement » ci-dessus, dans des conditions appropriées pour la croissance desdits microorganismes.

Par « **cultiver les microorganismes dans des conditions appropriées pour leur croissance** », on entend ici des méthodes de maintien et/ou de croissance des microorganismes vivants selon la présente invention.

L'homme du métier comprendra aisément que les conditions appropriées pour la croissance d'un microorganisme dépendront du microorganisme cultivé.

Ces conditions incluent par exemple des conditions de température, pH, agitation et d'aération (par exemple par apport en oxygène).

Les microorganismes sont de préférence cultivés sous température contrôlée. Ainsi, dans un mode de réalisation particulier, les microorganismes sont cultivés à l'étape b) à une température comprise entre 27°C et 30°C.

Les microorganismes sont de préférence cultivés sous pH contrôlé. Ainsi, dans un mode de réalisation particulier, les microorganismes sont cultivés à l'étape b) à un pH compris entre 6 et 7, de préférence à un pH de 6,5. De préférence, le pH est maintenu à un niveau constant pendant toute la durée de l'étape de culture. Le pH désiré peut être maintenu par toute méthode bien connue de l'homme du métier, telle que l'ajout d'acide ou de base.

Dans un mode de réalisation préféré, les microorganismes sont cultivés à l'étape b) à une température comprise entre 27°C et 30°C et à un pH compris entre 6 et 7, de préférence à un pH de 6,5.

Les microorganismes sont de préférence cultivés sous agitation. Les techniques d'agitation d'un milieu de culture sont bien connues de l'homme du métier. Dans un mode de réalisation particulier, les microorganismes sont cultivés à l'étape b) sous agitation par flux d'air filtré bouillonnant.

Les microorganismes sont de préférence cultivés sous aération contrôlée, par exemple sous apport d'oxygène. Les techniques d'aération, en particulier d'apport en oxygène, du milieu de culture, sont bien connues de l'homme du métier. Dans un mode de réalisation particulier, les microorganismes sont cultivés à l'étape b) sous un apport d'oxygène par flux d'air filtré, de préférence sous débit contrôlé. De préférence, les microorganismes sont cultivés à l'étape b) en présence de plus de 30% d'oxygène dissous.

Dans un mode de réalisation particulier, les microorganismes sont cultivés à l'étape b) en présence d'un anti-mousse.

De préférence, l'anti-mousse utilisé dans le cadre de l'invention est un anti-mousse de qualité alimentaire. Des anti-mousses de qualité alimentaire sont bien connus de l'homme du métier et incluent par exemple des anti-mousses basés sur des émulsions de silicone de type polydiméthylsiloxane. De préférence, un anti-mousse à base de silicone est utilisé, tel que l'anti-mousse CLEROL^{™} FBA 3107K commercialisé par PMC OUVRIE.

De préférence, les microorganismes sont cultivés à l'étape b) en conditions non-axéniques.

Par « **conditions non-axéniques** », on entend ici des conditions non-stériles.

La mise en œuvre de l'étape b) du procédé selon l'invention dans des conditions non-axéniques est particulièrement avantageuse car elle permet de réduire des surcoûts de fonctionnement tels que ceux liés à des obligations de stérilisation.

Les microorganismes peuvent être cultivés en milieu liquide, de manière continue, de manière semi-continue, ou en batch.

### Etape de collecte

L'étape c) du procédé selon l'invention consiste à collecter la biomasse obtenue à l'issue de l'étape b).

Par « **collecte de la biomasse** », on entend ici la récupération de la biomasse notamment par séparation du milieu de culture.

De préférence, la biomasse de microorganismes est collectée à l'étape c) lorsque la concentration en biomasse dans le milieu de culture a atteint au moins 10 g/L.

De manière également préférée, la biomasse de microorganismes est collectée à l'étape c) après au moins 42h de culture. De manière particulièrement préférée, la biomasse de microorganismes est collectée à l'étape c) après 42h de culture.

### Etapes supplémentaires optionnelles

Le procédé selon l'invention peut en outre comprendre, après l'étape c) de collecte, des étapes subséquentes de traitement de la biomasse collectée.

Ainsi, dans un mode de réalisation, le procédé selon l'invention comprend en outre, après l'étape c), une étape de filtration de ladite biomasse.

Par « **filtration** », on entend ici un procédé de séparation permettant de séparer les constituants d'un mélange qui possède une phase liquide et une phase solide au travers d'un milieu poreux. L'étape de filtration optionnelle du procédé selon l'invention vise à séparer la biomasse du milieu de culture.

Les techniques de filtration peuvent être séparées en techniques de filtration frontale et techniques de filtration tangentielle.

De préférence, ladite étape de filtration est une étape de filtration tangentielle.

De préférence, ladite étape de filtration est une étape de filtration à travers des filtres dont la taille des pores est de 300 kDa.

De manière particulièrement préférée, ladite étape de filtration est une étape de filtration tangentielle à travers des filtres dont la taille des pores est de 300 kDa.

Dans un mode de réalisation particulier, le procédé selon l'invention comprend en outre, après l'étape c), et éventuellement après l'étape de filtration, une étape de congélation et/ou de lyophilisation de la biomasse.

Par « **congélation** », on entend ici toute technique visant à faire passer un produit à l'état solide par des techniques de refroidissement forcé. Les techniques de congélation sont bien connues de l'homme du métier. De préférence, la biomasse est congelée à une température de -20°C.

Par « **lyophilisation** », on entend ici la dessiccation d'un produit préalablement congelé, par sublimation. Les techniques de lyophilisation sont bien connues du métier. De préférence, la biomasse est lyophilisée par traitement de 72h à -40°C et 250 µbar.

De manière particulièrement préférée, la biomasse collectée à l'étape c) est filtrée, puis congelée, de préférence à -20°C et séchée par lyophilisation, de préférence par traitement de 72h à -40°C et 250 µbar.

### Biomasse

La présente invention concerne également une biomasse de microorganismes du genre *Schizochytrium* ou *Aurantiochytrium,* telle que définie à la section « *Microorganismes du genre Schizochytrium ou Aurantiochytrium* » ci-dessus, susceptible d'être obtenue par le procédé de préparation tel que défini dans la section « *Procédé de préparation de* biomasse » ci-dessus.

Les présents inventeurs ont en effet montré qu'en produisant de la biomasse de microorganismes du genre *Schizochytrium* ou *Aurantiochytrium* avec le procédé de préparation décrit ci-dessus, il était possible d'obtenir une biomasse présentant des caractéristiques particulières, par rapport à une biomasse de microorganismes du genre *Schizochytrium* ou *Aurantiochytrium* obtenue au moyen d'un autre procédé. Cette biomasse présente en effet un profil lipidique très particulier et particulièrement avantageux pour une utilisation en alimentation des animaux d'élevage.

La présente invention a donc également pour objet une biomasse de microorganismes du genre *Schizochytrium* ou *Aurantiochytrium,* telle que définie à la section « *Microorganismes du genre Schizochytrium ou Aurantiochytrium* » ci-dessus, éventuellement susceptible d'être obtenue par le procédé de préparation tel que défini dans la section « *Procédé de préparation de biomasse* » ci-dessus, caractérisée en ce qu'elle présente le profil lipidique suivant :
- entre 55 et 65% d'acides gras totaux sous forme de lipides polaires, et
- au moins une des caractéristiques suivantes :
   - entre 18 et 25 µg d'acides gras totaux / mg de poids sec de biomasse,
   - entre 35 et 40% d'acide docosahexaénoïque (DHA) dans les lipides neutres,
   - un ratio DHA / acide docosapentaénoïque (DPA) dans les lipides neutres compris entre 4 et 4,5,
   - entre 45 et 55% de DHA dans les lipides polaires, et
   - un ratio DHA / DPA dans les lipides polaires compris entre 3 et 4.

Par « **acides gras totaux** », on entend ici la somme de tous les acides gras présents dans la biomasse. La quantité d'acides gras totaux est typiquement exprimée en poids par rapport au poids sec de biomasse.

La quantité d'acides gras totaux dans la biomasse peut être déterminée par toute technique bien connue de l'homme du métier. Typiquement, la quantité d'acides gras totaux dans la biomasse est déterminée comme décrit dans Soudant et al (2022) Sustainability 14:14573.

Dans un mode de réalisation particulier, la biomasse selon l'invention comprend entre 18 µg et 25 µg d'acides gras totaux par mg de poids sec de biomasse, de préférence entre 19 µg et 24 µg d'acides gras totaux par mg de poids sec de biomasse, de préférence encore entre 19,5 µg et 23 µg, entre 20 µg et 22 µg, ou entre 20,5 µg et 21,5 µg d'acides gras totaux par mg de poids sec de biomasse. De manière particulièrement préférée, la biomasse selon l'invention comprend entre 21 µg et 21,5 µg d'acides gras totaux par mg de poids sec de biomasse.

Les acides gras totaux incluent les acides gras venant des fractions lipidiques neutres et les acides gras venant des fractions lipidiques polaires.

Par « **lipide polaire** », on entend ici des lipides amphiphiles possédant une tête hydrophile et une queue hydrophobe. Les lipides polaires incluent typiquement les glycolipides et les phospholipides.

La quantité ou le pourcentage de lipides polaires parmi les acides gras totaux peut être déterminée par toute technique bien connue de l'homme du métier. Typiquement, la quantité ou le pourcentage de lipides polaires parmi les acides gras totaux est déterminée comme décrit dans Soudant et al (2022) Sustainability 14:14573.

La biomasse selon l'invention comprend avantageusement entre 55 et 65% d'acides gras totaux sous forme de lipides polaires, de préférence entre 60% et 64,5%, de préférence encore entre 61% et 64%, ou entre 62% et 63,5% d'acides gras totaux sous forme de lipides polaires. De manière particulièrement préférée, la biomasse selon l'invention comprend entre 63% et 63,5% d'acides gras totaux sous forme de lipides polaires.

Par « **lipide neutre** » ou « **lipide apolaire** », on entend ici des lipides ne comprenant que des groupements apolaires et lipophiles.

La quantité ou le pourcentage de lipides neutres parmi les acides gras totaux peut être déterminée par toute technique bien connue de l'homme du métier. Typiquement, la quantité ou le pourcentage de lipides neutres parmi les acides gras totaux est déterminée comme décrit dans Soudant et al (2022) Sustainability 14:14573.

Par « **acides gras oméga-3** » ou « **oméga-3** », on entend ici des acides gras polyinsaturés dont la première double liaison de la chaîne carbonée de l'acide, en comptant depuis l'extrémité opposée au carboxyle, est située sur la troisième liaison carbone-carbone. Les acides gras oméga-3 comprennent l'acide alpha-linolénique ou ω3α (18:3 ; ALA), l'acide eicosapentaénoïque (20:5 ; EPA) ou acide timnodonique ; et l'acide docosahexaénoïque (22:6 ; DHA) ou acide cervonique. Les lipides polaires sont généralement plus riches en acides gras oméga-3 que les lipides neutres qui contiennent principalement des acides gras saturés et monoinsaturés.

Par « **acide docosahexaénoïque** », « **DHA** » ou « **acide cervonique** », on entend ici un acide gras polyinsaturé oméga-3 de formule C₂₂H₃₂O₂.

La quantité ou le pourcentage de DHA parmi les lipides (polaires ou neutres) peut être déterminée par toute technique bien connue de l'homme du métier. Typiquement, la quantité ou le pourcentage de DHA parmi les lipides (polaires ou neutres) est déterminée comme décrit dans Soudant et al (2022) Sustainability 14:14573.

Dans un mode de réalisation particulier, la biomasse selon l'invention comprend avantageusement entre 35% et 40% d'acide docosahexaénoïque (DHA) dans les lipides neutres, de préférence entre 36% et 39%, de préférence entre 36% et 38% de DHA dans les lipides neutres. De manière particulièrement préférée, la biomasse selon l'invention comprend entre 36,5% et 37% de DHA dans les lipides neutres.

Dans un mode de réalisation particulier, la biomasse selon l'invention comprend entre 45% et 55% de DHA dans les lipides polaires, de préférence entre 46% et 54%, entre 47% et 53%, entre 48% et 52%, ou entre 49% et 51% de DHA dans les lipides polaires. De manière particulièrement préférée, la biomasse selon l'invention comprend entre 50% et 50,5% de DHA dans les lipides polaires.

Par « **acide docosapentaénoïque** » ou « **DPA** », on entend ici acide gras polyinsaturé à 22 atomes de carbone et cinq doubles liaisons (22:5) de formule CH₃(-CH₂)₂₀-COOH. Il se présente sous la forme de plusieurs isomères, notamment l'acide clupanodonique (tout-*cis-*Δ^{7,10,13,16,19} 22:5), un oméga-3 issu de l'élongation de l'acide eicosapentaénoïque, et l'acide d'Osbond (tout-*cis*-Δ^{4,7,10,13,16} 22:5), un oméga-6 issu de l'élongation de l'acide arachidonique.

La quantité ou le pourcentage de DPA parmi les lipides (polaires ou neutres) peut être déterminée par toute technique bien connue de l'homme du métier. Typiquement, la quantité ou le pourcentage de DPA parmi les lipides (polaires ou neutres) est déterminée comme décrit dans Soudant et al (2022) Sustainability 14:14573.

Dans un mode de réalisation particulier, la biomasse selon l'invention présente un ratio DHA / DPA dans les lipides neutres compris entre 4 et 4,5, de préférence entre 4,1 et 4,4. De manière particulièrement préférée, la biomasse selon l'invention présente un ratio DHA / DPA dans les lipides neutres compris entre 4,1 et 4,3.

Dans un mode de réalisation particulier, la biomasse selon l'invention présente un ratio DHA / DPA dans les lipides polaires compris entre 3 et 4, de préférence entre 3 et 3,7, de préférence encore entre 3,1 et 3,5. De manière particulièrement préférée, la biomasse selon l'invention présente un ratio DHA / DPA dans les lipides polaires compris entre 3,2 et 3,4.

De préférence, la biomasse selon l'invention présente le profil lipidique suivant :
- entre 55 et 65% d'acides gras totaux sous forme de lipides polaires, de préférence entre 60% et 64,5%, de préférence encore entre 61% et 64%, de préférence encore entre 62% et 63,5%, de manière particulièrement préférée, entre 63% et 63,5% d'acides gras totaux sous forme de lipides polaires, et
- au moins une, de préférence 2, 3, 4 ou 5 des caractéristiques suivantes :
   - entre 18 et 25 µg d'acides gras totaux / mg de poids sec de biomasse, de préférence entre 19 µg et 24 µg, de préférence encore entre 19,5 µg et 23 µg, entre 20 µg et 22 µg, ou entre 20,5 µg et 21,5 µg, de manière particulièrement préférée, entre 21 µg et 21,5 µg d'acides gras totaux par mg de poids sec de biomasse,
   - entre 35 et 40% d'acide docosahexaénoïque (DHA) dans les lipides neutres, de préférence entre 36% et 39%, de préférence entre 36% et 38% de DHA, de manière particulièrement préférée entre 36,5% et 37% de DHA dans les lipides neutres,
   - un ratio DHA / acide docosapentaénoïque (DPA) dans les lipides neutres compris entre 4 et 4,5, de préférence entre 4,1 et 4,4, de manière particulièrement préférée un ratio DHA / DPA dans les lipides neutres compris entre 4,1 et 4,3
   - entre 45 et 55% de DHA dans les lipides polaires, de préférence entre 46% et 54%, entre 47% et 53%, entre 48% et 52%, ou entre 49% et 51% de DHA, de manière particulièrement préférée entre 50% et 50,5% de DHA dans les lipides polaires, et
   - un ratio DHA / DPA dans les lipides polaires compris entre 3 et 4, de préférence entre 3 et 3,7, de préférence encore entre 3,1 et 3,5, de manière particulièrement préférée un ratio DHA / DPA dans les lipides polaires compris entre 3,2 et 3,4.

Dans un mode de réalisation préféré, la biomasse selon l'invention présente le profil lipidique suivant :
- entre 55 et 65% d'acides gras totaux sous forme de lipides polaires, de préférence entre 60% et 64,5%, de préférence encore entre 61% et 64%, de préférence encore entre 62% et 63,5%, de manière particulièrement préférée, entre 63% et 63,5% d'acides gras totaux sous forme de lipides polaires,
- entre 45 et 55% de DHA dans les lipides polaires, de préférence entre 46% et 54%, entre 47% et 53%, entre 48% et 52%, ou entre 49% et 51% de DHA, de manière particulièrement préférée entre 50% et 50,5% de DHA dans les lipides polaires, et

- éventuellement au moins une des caractéristiques suivantes :
   - entre 18 et 25 µg d'acides gras totaux / mg de poids sec de biomasse, de préférence entre 19 µg et 24 µg, de préférence encore entre 19,5 µg et 23 µg, entre 20 µg et 22 µg, ou entre 20,5 µg et 21,5 µg, de manière particulièrement préférée, entre 21 µg et 21,5 µg d'acides gras totaux par mg de poids sec de biomasse,
   - entre 35 et 40% d'acide docosahexaénoïque (DHA) dans les lipides neutres, de préférence entre 36% et 39%, de préférence entre 36% et 38% de DHA, de manière particulièrement préférée entre 36,5% et 37% de DHA dans les lipides neutres,
   - un ratio DHA / acide docosapentaénoïque (DPA) dans les lipides neutres compris entre 4 et 4,5, de préférence entre 4,1 et 4,4, de manière particulièrement préférée un ratio DHA / DPA dans les lipides neutres compris entre 4,1 et 4,3
   - un ratio DHA / DPA dans les lipides polaires compris entre 3 et 4, de préférence entre 3 et 3,7, de préférence encore entre 3,1 et 3,5, de manière particulièrement préférée un ratio DHA / DPA dans les lipides polaires compris entre 3,2 et 3,4.

Dans un mode de réalisation particulièrement préféré, la biomasse selon l'invention présente le profil lipidique suivant :
- entre 55 et 65% d'acides gras totaux sous forme de lipides polaires, de préférence entre 60% et 64,5%, de préférence encore entre 61% et 64%, de préférence encore entre 62% et 63,5%, de manière particulièrement préférée, entre 63% et 63,5% d'acides gras totaux sous forme de lipides polaires,
- entre 45 et 55% de DHA dans les lipides polaires, de préférence entre 46% et 54%, entre 47% et 53%, entre 48% et 52%, ou entre 49% et 51% de DHA, de manière particulièrement préférée entre 50% et 50,5% de DHA dans les lipides polaires,
- entre 18 et 25 µg d'acides gras totaux / mg de poids sec de biomasse, de préférence entre 19 µg et 24 µg, de préférence encore entre 19,5 µg et 23 µg, entre 20 µg et 22 µg, ou entre 20,5 µg et 21,5 µg, de manière particulièrement préférée, entre 21 µg et 21,5 µg d'acides gras totaux par mg de poids sec de biomasse,
- entre 35 et 40% d'acide docosahexaénoïque (DHA) dans les lipides neutres, de préférence entre 36% et 39%, de préférence entre 36% et 38% de DHA, de manière particulièrement préférée entre 36,5% et 37% de DHA dans les lipides neutres,
- un ratio DHA / acide docosapentaénoïque (DPA) dans les lipides neutres compris entre 4 et 4,5, de préférence entre 4,1 et 4,4, de manière particulièrement préférée un ratio DHA / DPA dans les lipides neutres compris entre 4,1 et 4,3, et
- un ratio DHA / DPA dans les lipides polaires compris entre 3 et 4, de préférence entre 3 et 3,7, de préférence encore entre 3,1 et 3,5, de manière particulièrement préférée un ratio DHA / DPA dans les lipides polaires compris entre 3,2 et 3,4.

### Utilisation comme ingrédient pour aliment et aliment

La présente invention concerne également l'utilisation de la biomasse de microorganismes du genre *Schizochytrium* ou *Aurantiochytrium* telle que définie dans la section « Biomasse » ci-dessus, comme ingrédient dans un aliment pour animaux d'élevage.

La présente invention concerne également un aliment pour animaux d'élevage, comprenant entre 15% et 30% en poids de biomasse de microorganismes du genre *Schizochytrium* ou *Aurantiochytrium* telle que définie dans la section « Biomasse » ci-dessus.

Par « **aliment** », on entend ici une substance ou un produit, transformé, partiellement transformé ou non transformé, destiné à être consommé par des animaux pour ses propriétés nutritives ou pour le plaisir.

Par « **animaux d'élevage** », on entend ici n'importe quel animal domestiqué élevé dans des infrastructures agricoles ou industrielles et n'importe quel animal aquatique élevé dans des infrastructures d'aquaculture. Dans un mode de réalisation particulier, l'animal d'élevage est ainsi un animal d'aquaculture, en particulier un animal de pisciculture. Les animaux de pisciculture incluent par exemple l'esturgeon, la carpe, le gardon, le brochet, le cabillaud, le bar, la dorade, le maigre, la perche, le sandre, le tilapia, le turbot, l'ombre, le saumon, la truite, ou le silure.

Par « **aliment pour animaux d'élevage** », on entend ici un aliment, tel que défini ci-dessus, adapté et destiné à l'alimentation des animaux d'élevage tels que définis ci-dessus.

Dans un mode de réalisation particulier, l'aliment pour animaux d'élevage selon l'invention est un aliment pour animal d'aquaculture, tel que défini ci-dessus, ou aliment aquacole.

Par « **aliment aquacole** », on entend ici un aliment adapté et destiné à l'alimentation d'animaux d'aquaculture, en particulier d'animaux de pisciculture.

L'aliment aquacole peut être utilisé pour l'alimentation de tout type de poisson d'élevage, tels que l'esturgeon, la carpe, le gardon, le brochet, le cabillaud, le bar, la dorade, le maigre, la perche, le sandre, le tilapia, le turbot, l'ombre, le saumon, la truite, ou le silure.

Dans un mode de réalisation particulier, l'aliment selon l'invention est un aliment pour bar, en particulier pour bar d'élevage, en particulier un aliment pour juvénile de bar.

Par « **juvénile de poisson** », on entend ici le stade de développement d'un poisson qui précède la période adulte, plus particulièrement un jeune poisson qui n'est pas encore apte à se reproduire.

L'aliment pour animaux d'élevage selon l'invention, en particulier l'aliment aquacole, plus particulièrement l'aliment pour bar d'élevage, encore plus particulièrement l'aliment pour juvénile de bar, comprend entre 15% et 30% en poids de biomasse de microorganismes du genre *Schizochytrium* ou *Aurantiochytrium* telle que définie dans la section « Biomasse » ci-dessus.

Dans un mode de réalisation particulier, l'aliment pour animaux d'élevage, en particulier l'aliment aquacole, plus particulièrement l'aliment pour bar d'élevage, encore plus particulièrement l'aliment pour juvénile de bar, comprend entre 20% et 25% en poids de biomasse de microorganismes du genre *Schizochytrium* ou *Aurantiochytrium* telle que définie dans la section « *Biomasse* » ci-dessus.

De manière particulièrement préférée, l'aliment pour animaux d'élevage, en particulier l'aliment aquacole, plus particulièrement l'aliment pour bar d'élevage, encore plus particulièrement l'aliment pour juvénile de bar, comprend 15% en poids de biomasse de microorganismes du genre *Schizochytrium* ou *Aurantiochytrium* telle que définie dans la section « Biomasse » ci-dessus.

L'aliment pour animaux d'élevage selon l'invention, en particulier l'aliment aquacole, plus particulièrement l'aliment pour bar d'élevage, encore plus particulièrement l'aliment pour juvénile de bar, peut en outre comprendre tout ingrédient adapté à l'alimentation d'animaux d'élevage, en particulier de poissons d'élevage, plus particulièrement de bar d'élevage, encore plus particulièrement de juvénile de bar.

Des exemples d'ingrédients adaptés à l'alimentation d'animaux d'élevage, en particulier de poissons d'élevage, plus particulièrement de bar d'élevage, encore plus particulièrement de juvénile de bar sont bien connus de l'homme du métier et incluent les ingrédients issus de la pêche minotière, tels que les farines marines ; les hydrolysats de protéines marines ; les huiles de poisson ; les céréales, en particulier les céréales à faible digestibilité telles que la farine de blé ; les légumineuses ; et le gluten de blé.

Par « **ingrédient issu de la pêche minotière** », on entend ici un ingrédient issu de la pêche spécialisée dans la capture d'espèces transformées en farines, ou en huiles par les usines de réduction. Des exemples d'espèces utilisées dans le cadre de la pêche minotière incluent les petits poissons pélagiques tels que l'anchois péruvien, le capelan et le merlan bleu, ainsi que le krill.

Par « **krill** », on entend ici de petits crustacés des eaux froides, de l'ordre des *Euphausiacea.*

Dans un mode de réalisation préféré, l'aliment pour animaux d'élevage selon l'invention, en particulier l'aliment aquacole, plus particulièrement l'aliment pour bar d'élevage, encore plus particulièrement l'aliment pour juvénile de bar, comprend moins de 50 % en poids d'ingrédient issu de la pêche minotière, en particulier de farines marines, issues de poissons et/ou de krill, et/ou d'hydrolysats de protéines marines, de préférence moins de 49% en poids d'ingrédient issu de la pêche minotière, en particulier de farines marines, issues de poissons et/ou de krill, et/ou d'hydrolysats de protéines marines.

Par « **céréales à faible digestibilité** », on entend ici des céréales présentant un faible degré de digestibilité chez les animaux auxquels les céréales sont administrées. Ainsi, dans un mode de réalisation, les céréales à faible digestibilité sont des céréales à faible digestibilité pour des poissons d'élevage, en particulier pour des bars d'élevage, plus particulièrement pour des juvéniles de bar.

Par « **digestibilité** », on entend ici le degré auquel une matière organique est digérée par un animal. Ce qui est ingéré, l'ingesta est corrélé aux fécès permettant de définir le coefficient d'utilisation digestive (CUD) entre la matière organique utile et celle inutile ou peu digérée. Par digestibilité, on entend ici la digestibilité apparente (égale à la quantité ingérée moins la quantité excrétée dans les fécès, sur la quantité ingérée) ou la digestibilité réelle (qui correspond à la proportion d'aliments qui disparait réellement dans le tube digestif). Les techniques permettant de déterminer le degré de digestibilité d'une céréale sont bien connues de l'homme du métier et sont par exemple décrites dans Choubert et al. (1982) Aquaculture 29:185-189.

Par « **faible digestibilité** », on entend ici une digestibilité apparente ou réelle inférieure à 60%.

Les céréales à faible digestibilité utilisées dans l'aliment selon la présente invention incluent par exemple le blé, en particulier sous forme de farines de blé, de graines entières, de son de blé ou de remoulage de blé.

Dans un mode de réalisation préféré, l'aliment pour animaux d'élevage selon l'invention, en particulier l'aliment aquacole, plus particulièrement l'aliment pour bar d'élevage, encore plus particulièrement l'aliment pour juvénile de bar comprend moins de 10% en poids de céréales à faible digestibilité, en particulier de blé, plus particulièrement sous forme de farine de blé, de préférence moins de 9% en poids de céréales à faible digestibilité, en particulier de blé, plus particulièrement sous forme de farine de blé.

Dans un mode de réalisation préféré, l'aliment pour animaux d'élevage selon l'invention, en particulier l'aliment aquacole, plus particulièrement l'aliment pour bar d'élevage, encore plus particulièrement l'aliment pour juvénile de bar comprend :
- entre 15% et 30% en poids, de préférence 15% en poids, de biomasse de microorganismes du genre *Schizochytrium* ou *Aurantiochytrium* telle que définie dans la section « *Biomasse* » ci-dessus,
- entre 40% et 50% en poids, de préférence entre 48% et 49% en poids d'ingrédient issu de la pêche minotière, en particulier de farines marines, issues de poissons et/ou de krill, et/ou d'hydrolysats de protéines marines,
- entre 7% et 9% en poids d'huile de poisson,
- entre 0 et 0,5% en poids de lécithine de soja,
- entre 11% et 13% en poids de gluten de blé,
- entre 7% et 9% en poids, de préférence entre 7% et 8% en poids, de céréales à faible digestibilité, en particulier de blé, plus particulièrement sous forme de farine de blé, et
- entre 2% et 5% en poids d'amidon de pois.

L'aliment pour animaux d'élevage selon l'invention peut être sous toute forme adaptée aux animaux auxquels il est destiné. Ainsi, lorsque l'aliment est un aliment aquacole, il peut être sous forme de granulés, en particulier sous forme de granulé extrudé ou de granulé pressé, sous forme de miette, d'émulsion, de comprimé ou de semoulette.

### Méthode d'élevage de poissons

La présente invention concerne également une méthode d'élevage de poissons, comprenant la fourniture de la biomasse de microorganismes du genre *Schizochytrium* ou *Aurantiochytrium* telle que définie dans la section « Biomasse » ci-dessus, auxdits poissons.

Les poissons élevés dans la méthode d'élevage selon la présente invention sont de préférence des poissons de pisciculture.

Les animaux de pisciculture incluent par exemple l'esturgeon, la carpe, le gardon, le brochet, le cabillaud, le bar, la dorade, le maigre, la perche, le sandre, le tilapia, le turbot, l'ombre, le saumon, la truite, ou le silure.

De préférence, les poissons élevés dans la méthode d'élevage selon la présente invention sont des bars, de préférence des juvéniles de bars.

La biomasse de microorganismes du genre *Schizochytrium* ou *Aurantiochytrium* telle que définie dans la section « *Biomasse* » ci-dessus, peut être fournie aux poissons sous toute forme appropriée pour l'alimentation de poissons. Dans un mode de réalisation préférée, la biomasse de microorganismes du genre *Schizochytrium* ou *Aurantiochytrium* telle que définie dans la section « *Biomasse* » ci-dessus est sous la forme de l'aliment tel que défini dans la section « *Utilisation comme ingrédient pour aliment et aliment* » ci-dessus.

La biomasse, de préférence sous forme de l'aliment tel que défini dans la section *« Utilisation comme ingrédient pour aliment et aliment* » ci-dessus, peut être fournie aux poissons par toute technique appropriée pour l'alimentation de poissons.

La biomasse, de préférence sous forme de l'aliment tel que défini dans la section *« Utilisation comme ingrédient pour aliment et aliment* » ci-dessus, est typiquement fournie aux poissons pendant une durée appropriée pour l'élevage des desdits poissons. Ainsi, dans un mode de réalisation particulier, la biomasse, de préférence sous forme de l'aliment tel que défini dans la section « *Utilisation comme ingrédient pour aliment et aliment* » ci-dessus, est fournie aux poissons pendant une durée supérieure ou égale à 5 semaines, de préférence encore pendant une durée de 5 à 10 semaines, en particulier de 6 à 9 semaines, de manière particulièrement préférée de 7 à 8 semaines.

La biomasse, de préférence sous forme de l'aliment tel que défini dans la section *« Utilisation comme ingrédient pour aliment et aliment* » ci-dessus, est typiquement fournie aux poissons à une fréquence appropriée pour l'élevage des desdits poissons. Ainsi dans un mode de réalisation particulier, la biomasse, de préférence sous forme de l'aliment tel que défini dans la section « *Utilisation comme ingrédient pour aliment et aliment* » ci-dessus, est fournie aux poissons tous les jours.

La biomasse, de préférence sous forme de l'aliment tel que défini dans la section *« Utilisation comme ingrédient pour aliment et aliment* » ci-dessus, est typiquement fournie aux poissons à une dose appropriée pour l'élevage des desdits poissons. Ainsi, dans un mode de réalisation particulier, la biomasse, de préférence sous forme de l'aliment tel que défini dans la section « *Utilisation comme ingrédient pour aliment et aliment* » ci-dessus, est fournie aux poissons à une dose moyenne journalière de 2% à 3% du poids vif.

Dans un mode de réalisation particulièrement préféré, la biomasse, de préférence sous forme de l'aliment tel que défini dans la section « *Utilisation comme ingrédient pour aliment et aliment* » ci-dessus, est fournie aux poissons, en particulier aux bars d'élevage, plus particulièrement aux juvéniles de bar, pendant une durée de 5 à 10 semaines, de préférence une durée de 7 semaines, de préférence tous les jours, préférentiellement à une dose moyenne journalière de 2% à 3% du poids vif.

La présente invention sera illustrée plus en détails par les exemples ci-dessous.

### EXEMPLES

### EXEMPLE 1: Préparation de biomasse de A. mangrovei

### 1. Procédé d'obtention de la biomasse de A. mangrovei

### 1.1. Préparation de l'inoculum A. mangrovei

L'inoculation initiale a été réalisée à l'aide de 2 ml d'une culture principale d' A. *mangrovei* (RCC893) à une concentration de 1 × 10⁷ cellules/ml.

Des ballons de 500 mL ont été préalablement inoculés dans 250 mL de milieu « Yeast Extract Peptone » (YEP) contenant 15 g/L de sel marin (SigmaTM, Saint Louis, MO, USA ; Sigma S9883), 2 g/L de peptone de caséine (VWRTM Rosny-sous-bois, France, 84610. 0500), 2 g/L d'extrait de levure (VWRTM Rosny-sous-bois, France, 84601.5000) et 20 g/L de glucose (TITOL chimica SpATM, Pontecchio Polesine, Italie, Glucose anhydre pur CA:50-99-7).

La culture a ensuite été maintenue sur une plateforme d'agitation (100 rpm) pendant 48 h à 23-25°C. Ensuite, 2 mL de la première culture ont été utilisés pour inoculer quatre nouveaux ballons dans les mêmes conditions pendant 64 h. Le contenu des quatre cultures a été transféré dans 4 carboys de 20 L pour inoculer 8 L de milieu YEP dans chacun d'eux. Les Carboys ont été maintenues pendant 24 h sur la plate-forme d'agitation (100 rpm), avec un apport d'air (4,8 L/min à travers un tuyau de 4 mm de diamètre). Après contrôle qualité, les 4 cultures de 8 L ont finalement été utilisées pour l'inoculation de deux cylindres de 800 L en poly-méthyl-méthacrylate (PMMA) pour culture en batch (500 L).

### 1.2. Préparation du milieu de culture

Le milieu pour un batch final de 500L est composé de:
1) Azote - (NH₄)₂SO₄ (LORIAL-MOLSHEIM): 10,81g/L
2) Substrat carboné - Isosweet 470 (UNIVAR): 48,19g/L
3) Solution N°1 métaux trace (1L) :
   - CoCl₂, 6H₂0 (PROLABO-PARIS) :11,87mg/L
   - CuSO,H₂O (Panreac Applicherm-Germany-7758-99-8) : 37,66g/L
   - H₃BO₃ (Panreac Applicherm-Germany-10043-35-3 ): 4,84g/L
   - MnCl₂, 4H₂O (Honeywell Fluka-Germany-13446-34-9) : 2,24g/L
   - Na₂MoO₄, 2H₂O (Merck-7631-95-0) : 29,29mg/L
   - Na₂SeO₃ (Fisher Scientific-UK-1010218-8) : 11,30mg/L
   - NiSO₄, 6H₂O(MERCK-10101-97-0) : 8,96mg/L
   - ZnSO₄, 7H₂O (Sigma Aldrich-Germany-7446-20-7) : 33,01g/L
4) Solution N°2 métaux trace (0,25L) :
   - FeSO₄, 7H₂O (Sigma Aldrich-Germany-7782-63-0) : 81,79g/L
5) Solution N°3 Vitamine (50mL) :
   - Vitamine B9-Folate Totaux (Alfa Aesar-Germany-75708-92-8) : 8,12g/L dissous dans Eau Milli Q +NaOH 0,1N
6) Solution N°4 Vitamines (1L) :
   - Vitamine B3-Niacine (Sigma Aldrich-Germany-8-18714-1000) : 11g/L
7) Solution N°5 Vitamine (1L) :
   - Vitamine C - Acide Ascorbique (Acros Organics-China-5081-7) : 75,2g/L
   - Vitamine B8 - Biotine (Alfa Aesar-Germany-58-85-5) : 20,5mg/L
   - Vitamine B5 - Acide Pantothénique (Alfa Aesar-Germany-137-08-6) : 4,24g/L
   - Vitamine B12 (Sigma Aldrich-St Louis-USA-68-19-9) : 13 mg/L
   - Vitamine B1 - Thiamine (Panreac Applicherm-Germany-6703-8) : 208mg/L
8) Solution N°6 Vitamine (125mL) :
   - Vitamine B6 - Pyridoxine (Alfa Aesar-Germany-58-56-0): 13,28g/L

Les solutions 1 et 2 ainsi que les Carboys contenant l'ISOSWEET 470 ont été autoclavés (121°C, 20 min, 1 bar) et les solutions 3-4-5-6 ont été filtrées à 0,2 µm sous hotte à flux laminaire.

L'azote sous forme de poudre a été directement mélangé dans la saumure 6% d'extrait sec (ES) avant d'être solubilisé grâce à l'action d'une pompe centrifuge (pompe VITACHROM VC d'une puissance de 5,5 kw, faisant partie du pilote de filtration SIVA^{™} mentionné ci-dessous) qui fonctionne en boucle fermée à un débit de 2,5 m³/h pendant environ 45 min.

La saumure utilisée est un coproduit 6% ES est un coproduit de l'industrie laitière, issu du traitement de déminéralisation du « petit-lait ». Ce produit est riche en sels, mais également en phosphore. Or cet élément est indispensable à l'agriculture mais n'est pas en quantité illimitée à l'échelle mondiale. Le procédé décrit ici permet de le valoriser au mieux. Enfin, la saumure 6% ES apporte l'eau, élément indispensable à toute forme de vie. Le procédé s'avère donc également particulièrement vertueux en termes de gestion de l'eau.

Une fois l'azote ammoniacal ((NH₄)₂SO₄) solubilisé dans la saumure, l'ensemble a été filtré grâce au pilote de filtration SIVA^{™} qui se compose de deux cartouches comportant chacune 11 canaux (diamètre interne de 4,6 mm) ce qui représente une surface totale de 3,5 m² de membrane céramique avec une porosité de 300 kDa. Les conditions de filtration étaient les suivantes : 4 m/s pour la vitesse du retentât sur la membrane, 2500 L/h pour le taux de recirculation du retentât, 0,6 à 0,9 bars pour la pression transmembranaire, 300 à 180 L/h pour le débit du perméat.

Le mélange saumure et azote a ainsi été transféré de façon alternée dans chacun des 2 cylindres en PMMA qui constituent le réacteur. Tous les autres intrants nécessaires ont été introduits manuellement au sommet des deux cylindres. Ainsi les solutions 1 à 6 ont été ajoutées manuellement aux cylindres puis les Carboys d'ISOSWEET 470 ont été ajoutés. Le volume total a enfin été ajusté à 500L avec le mélange saumure 6% ES et azote ammoniacal.

### 1.3. Préparation de la biomasse de A. mangrovei

### a) Gestion des paramètres de culture

La température de la culture a été régulée entre 27°C et 30°C par l'immersion d'un chauffage électrique en acier inoxydable (2 kW pour 500 L) tandis que le pH a été maintenu automatiquement à 6,5 par l'ajout régulier d'une solution de NaOH 30% (WWR-28217.361) à l'aide d'une sonde pH de la marque XYLEM (STM-20522747-11-EGAT150VP-X-SONDE PH)

Deux cylindres de 800 L fabriqués en PMMA ont été utilisés pour produire environ 10 kg de biomasse dans des conditions non stériles. L'eau (pour le processus et le nettoyage) a été fournie par une pompe et acheminée au sommet des cylindres par une buse rotative. L'agitation et l'apport d'O₂ dans chaque cylindre ont été assurés par un flux d'air filtré (0,2 µm) bouillonnant depuis le bas du cylindre, par le biais d'un bulleur situé en fond de cuve et intégré dans la platine d'un diamètre de 340 mm perforée de trous de 2 mm positionnés en quinconce tous les 10 mm (environ 500 trous). Le débit d'air a été contrôlé par un débitmètre massique (Airlitec-MCF0151AGND010000)

Les deux cylindres de 800 L ont été remplis avec 500 L de milieu de culture et chacun a été inoculé avec 16 L d'inoculum tel que préparé ci-dessus. Un anti-mousse (de qualité alimentaire) à base de silicone a été ajouté (concentration finale : 1 mL.L⁻¹, CLEROL FBA 3107K - PMC OUVRIE-Carvin). Les microorganismes *A*. *mangrovei* ont ensuite été cultivés en conditions non axéniques pendant 42 h. Au terme de cette durée de culture, la concentration atteinte de biomasse était d'environ 10 g/L dans les deux cylindres.

### b) Récolte de la biomasse

Les cultures des deux cylindres (1000 L) ont été regroupées et filtrées à l'aide d'un outil de filtration tangentielle SIVA^{™}. Pendant la filtration tangentielle, des échantillons ont été prélevés toutes les heures afin de contrôler la composition en acides gras, notamment leur teneur en AGPI-LC n-3. Le volume final du retentât était d'environ 100 L à 90 g/L (équivalent Poids sec) de biomasse.

La biomasse récoltée a été congelée à -20 °C avant d'être séchée en aval par lyophilisation en externe par Eurolyo (72 h à -40 °C et 250 µbar).

### 2. Caractérisation de la biomasse obtenue

### 2.1. Concentration cellulaire et matière sèche

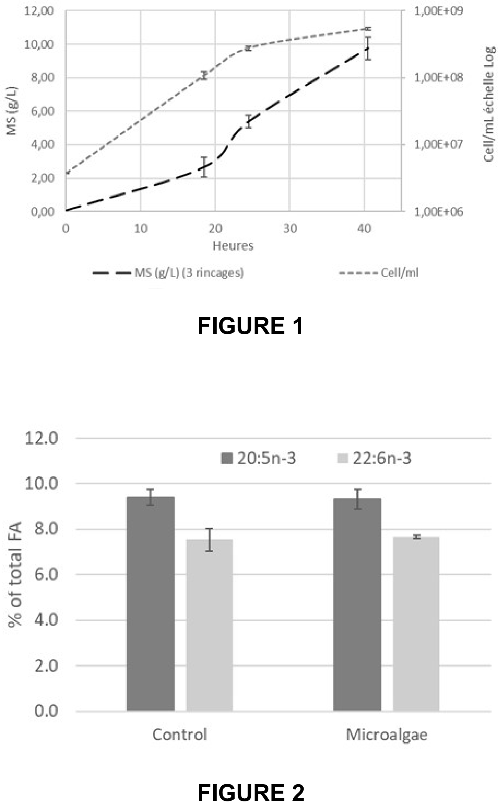

La **Figure** 1 présente la cinétique de croissance de *A*. *mangrovei* pendant la culture. Elle montre que la composition du milieu et les conditions de culture décrites ci-dessus permettent d'obtenir près de 10g/L de matière sèche en 42 h.

### 2.2. Compositions proximale et lipidique de la biomasse produite

Les compositions proximale et lipidique ont été analysées sur trois échantillons de biomasse de *A*. *mangrovei* produite comme décrit ci-dessus.

**Tableau 1 : Composition proximale et composition en acides gras de la biomasse selon l'invention**

| | Biomasse | |
|---|---|---|
| | Moyenne | Ecart-type |
| % humidité | 7,3 | 0,2 |
| % cendres | 24 ;4 | 0,1 |
| % protéines (estimation par analyseur CHN) | 47,6 | 0,2 |
| % lipides | 12,6 | 0,8 |
| Acides gras totaux (µg) / mg de poids sec | 21,1 | 1,9 |
| % d'acides gras totaux sous forme de lipides neutres | 40,0 | 1,7 |
| % de DHA dans les lipides neutres | 36,9 | 0,6 |
| DHA/DPA dans les lipides neutres | 4,2 | 0,1 |
| % de DHA dans les lipides polaires | 50,2 | 1,0 |
| DHA/DPA dans les lipides polaires | 3,3 | 0,1 |

Les mesures biochimiques de composition en acides aminés ont été réalisées sur des biomasses *de Schizochytrium* commercial (DHA Gold commercialisé par la société DSM) lyophilisées et exprimées par poids sec (contenant environ 7% d'eau).

**Tableau 2 : Aminogramme d'une biomasse de Schizochytrium commercial**

| Acides aminés | Total acides aminés (g) / 100g | |
|---|---|---|
| | Moyenne | Ecart-type |
| Acide aspartique | 3,1 | 0,25 |
| Thréonine | 1,64 | 0,13 |
| Sérine | 1,62 | 0,13 |
| Acide glutamique | 4,84 | 0,39 |
| Proline | 1,15 | 0,09 |
| Glycine | 1,51 | 0,12 |
| Alanine | 1,86 | 0,15 |
| Cystine | 0,5 | 0,04 |
| Valine | 1,55 | 0,12 |
| Méthionine | 0,56 | 0,04 |
| Isoleucine | 1,27 | 0,10 |
| Leucine | 2,07 | 0,17 |
| Tyrosine | 1,05 | 0,08 |
| Phénylalanine | 1,23 | 0,10 |
| Histidine | 0,66 | 0,05 |
| Lysine | 1,9 | 0,15 |
| Arginine | 2,19 | 0,18 |
| TOTAL | 28,66 | 2,29 |

### EXEMPLE 2 : Formulation d'un aliment aquacole mettant en œuvre la biomasse produite à l'exemple 1.

Deux aliments aquacoles pour juvéniles de bar d'un mois ont été préparés. Le premier aliment est un aliment témoin. Le deuxième est un aliment contenant 15% (en équivalent poids sec) de la biomasse d'*A*. *mangrovei* préparée et caractérisée à l'exemple 1.

La formulation détaillée de ces deux aliments est présentée dans le tableau 3.

**Tableau 3 : Formulation de l'aliment témoin et de l'aliment selon l'invention**

| Ingrédient (g/100 g d'aliment) | Aliment témoin | Aliment selon l'invention |
|---|---|---|
| Biomasse d'*A*. *mangrovei* | 0,0 | 15,0 |
| Farines marines (poisson, krill) | 53,0 | 48,3 |
| Hydrolysats de protéines marines | 5,0 | 0,0 |
| Huile de poisson | 8,5 | 8,6 |
| Lécithine de soja (95 acétone insoluble) | 0,9 | 0,1 |
| Gluten de blé | 11,5 | 12,7 |
| Farine de blé | 16,4 | 8,0 |
| Amidon de pois | 2,0 | 4,6 |
| Solide (acides aminés, additif, ...) | 2,7 | 2,7 |
| PROTEINE BRUTE | 52 | 52 |
| MATIERE GRASSSE BRUTE | 16 | 16 |
| TOTAL PHOSPHOLIPIDES | 3,00 | 3,24 |
| DHA/EPA | 0,69 | 1,14 |
| CENDRES BRUTES | 9,1 | 8,6 |
| CALCIUM | 1,8 | 1,9 |
| PHOSPHORE TOTAL | 1,3 | 1,3 |

Les deux aliments sont très similaires en termes d'apports (iso-énergie, isoprotéique...). C'est un point important qui permet une comparaison objective des différences qualitatives de ces deux aliments.

Ainsi concernant l'apport spécifique en acides aminés essentiels (voir tableau 4), les calculs matriciels permettent de mettre en évidence une similitude de composition malgré le fait qu'une partie des farines de poisson est substituée par la biomasse d'A. *mangrovei.*

**Tableau 4 : Proportion d'acides aminés essentiels pour 100g d'aliment.**

| (g / 100 g d'aliment) | Aliment témoin | Aliment selon l'invention |
|---|---|---|
| Protéines brutes | 52,0 | 51,7 |
| Lysine | 3,6 | 3,4 |
| Méthionine Cystine | 2,1 | 1,9 |
| Arginine | 2,8 | 2,7 |
| Thréonine | 2,1 | 2,0 |
| Tryptophane | 0,5 | 0,4 |
| Leucine | 3,6 | 3,4 |
| Isoleucine | 2,1 | 2,0 |
| Valine | 2,5 | 2,3 |

Les contenus et compositions en acides gras des deux aliments ont été analysés selon la méthode décrite dans Soudant et al 2022. Les résultats sont présentés dans le tableau 5.

**Tableau 5 : Contenus en acides gras totaux en g par 100 g d'aliment sec des aliments expérimentaux. Comparaison des valeurs issus des calculs matriciels et des analyses réalisées.**

| (g / 100 g d'aliment) | Valeurs calculées | | Valeurs mesurées | |
|---|---|---|---|---|
| | Aliment témoin | Aliment selon l'invention | Aliment témoin | Aliment selon l'invention |
| C16:0 acide palmitique | 2,4 | 2,4 | 2,0 | 1,9 |
| C18:0 acide stéarique | 0,5 | 0,5 | 0,5 | 0,4 |
| C18:1 acide oléique | 1,8 | 1,8 | 1,4 | 1,2 |
| C18:2 (n-6) acide linoléique | 0,6 | 0,5 | 0,7 | 0,5 |
| C18:3 (n-3) acide α-linolénique | 0,2 | 0,2 | 0,1 | 0,1 |
| C20:4 (n-6) acide arachidonique | 0,2 | 0,2 | 0,1 | 0,1 |
| Total acides gras saturés | 4,5 | 4,4 | 3,3 | 3,1 |
| Total acides insaturés | 8,3 | 8,1 | 7,6 | 6,9 |
| Total acides gras polyinsaturés | 4,7 | 4,6 | 4,2 | 3,9 |
| Total acides gras monoinsaturés | 0,0 | 0,0 | 3,3 | 3,0 |
| Total (n-3) | 3,1 | 3,2 | 3,1 | 2,9 |
| Total (n-6) | 0,8 | 0,7 | 1,0 | 0,8 |
| C20:5 (n3) EPA | 1,6 | 1,6 | 1,4 | 1,4 |
| C22:6 (n-3) DHA | 1,1 | 1,2 | 0,9 | 0,9 |
| EPA+DHA (C20:5 + C22:6) | 2,6 | 2,7 | 2,3 | 2,3 |
| (n-3) / (n-6) | 3,9 | 4,3 | 3,0 | 3,6 |
| Total phospholipides | 1,4 | 1,5 | 1,5 | 1,1 |

En moyenne les valeurs mesurées sont à 82% et 90% de celles des teneurs calculées, respectivement pour l'aliment contenant la biomasse selon l'invention et l'aliment témoin. On peut noter que le ratio (n-3)/n-6) est nutritionnellement plus favorable dans l'aliment contenant la biomasse selon l'invention.

Au-delà du fait que le ratio (n-3) /(n-6) est plus intéressant dans l'aliment contenant la biomasse selon l'invention, il apparait également que les pourcentages de DHA et d'EPA dans les acides gras totaux sont plus importants dans l'aliment avec la biomasse selon l'invention (voir tableau 6).

**Tableau 6 : Composition en acides gras exprimée en % des acides gras totaux des aliments témoin et selon l'invention**

| % des lipides | Valeurs mesurées | |
|---|---|---|
| | Aliment témoin | Aliment selon l'invention |
| C16:0 acide palmitique | 18,3 | 18,8 |
| C18:0 acide stéarique | 4,1 | 4,1 |
| C18:1 acide oléique | 12,5 | 11,9 |
| C18:2 (n-6) acide linoléique | 6,8 | 5,3 |
| C18:3 (n-3) acide α-linolénique | 1,1 | 1,0 |
| C20:4 (n-6) acide arachidonique | 1,2 | 1,3 |
| Total acides gras saturés | 29,8 | 30,7 |
| Total acides insaturés | 68,5 | 67,5 |
| Total acides gras polyinsaturés | 38,5 | 38,4 |
| Total acides gras monoinsaturés | 30,0 | 29,2 |
| Total (n-3) | 28,0 | 29,0 |
| Total (n-6) | 9,2 | 8,0 |
| C20:5 (n3) EPA | 12,9 | 13,5 |
| C22:6 (n-3) DHA | 8,3 | 8,7 |
| EPA+DHA (C20:5 + C22:6) | 21,2 | 22,2 |
| (n-3) / (n-6) | 3,0 | 3,6 |

Enfin il est intéressant de noter que les acides gras polyinsaturés (AGPI) se répartissent différemment entre les lipides neutres et les lipides polaires. Le DHA est en proportion supérieure dans les lipides polaires tandis que c'est l'inverse pour l'EPA pour les deux aliments (voir tableau 7).

**Tableau 7 : Pourcentage en EPA (20:5n-3) et en DHA (22:6n-3) des lipides neutres et des lipides polaires des aliments témoins et selon l'invention.**

| Aliments | Aliment témoin | | Aliment selon l'invention | |
|---|---|---|---|---|
| | Moyenne | Ecart-type | Moyenne | Ecart-type |
| % d'EPA dans les lipides neutres | 13,8 | 0,2 | 13,9 | 0,1 |
| % d'EPA dans les lipides polaires | 8,0 | 0,2 | 8,1 | 0,1 |
| % de DHA dans les lipides neutres | 8,0 | 0,1 | 8,1 | 0,1 |
| % de DHA dans les lipides polaires | 11,7 | 0,2 | 16,6 | 0,4 |

Le % de DHA dans les lipides polaires des aliments avec la biomasse selon l'invention est autour de 16% et est inférieur à 12% dans l'aliment témoin. Un plus grand apport d'acides gras polyinsaturés sous forme de phospholipides par la biomasse selon l'invention peut contribuer à une meilleure assimilation de ces derniers par les juvéniles de bar.

### EXEMPLE 3 : Utilisation de l'aliment aquacole de l'exemple 2 dans l'alimentation de juvéniles de bar

Une période de « pré-grossissement » d'un mois avec un aliment commercial (Néo Start Loop 1 commercialisé par Le Gouessant Aquaculture) a permis à des juvéniles de bar de croître de 1,5 g à presque 4,0 g. Arrivés à cette taille, les juvéniles de bar ont alors été nourris pendant 7 semaines avec les 2 aliments (témoin et selon l'invention) décrits dans l'exemple 2.

Chaque conditionnement nutritionnel a été réalisé en triplicas (3 bacs par condition).

### 1. Effet sur les contenus et compositions en acides gras des muscles de juvéniles de bar.

Au terme de l'expérimentation, trois juvéniles dans chaque bac (soit 9 juvéniles par condition nutritionnelle) ont été prélevés et disséqués pour les analyses lipidiques dans le muscle et le foie. Les tissus ont été lyophilisés et extraits comme décrit dans Soudant et al. 2022.

Comme montré dans le tableau 8, les muscles des bars nourris avec l'aliment selon l'invention tendent à contenir moins de lipides que ceux nourris avec l'aliment témoin. Cette tendance reflète notamment un niveau de lipides de réserve plus faible (20,5 µg/mg de poids sec avec l'aliment selon l'invention par rapport à 28,1 µg/mg de poids sec avec l'aliment témoin). Cette teneur plus faible en lipide de réserve est bénéfique à la fois pour la physiologie du poisson mais également à terme pour le consommateur.

**Tableau 8 : Contenu en acides gras totaux (AGT) dans le muscle et leur répartition entre les lipides neutres et les lipides polaires**

| Aliments | Aliment témoin | | Aliment selon l'invention | |
|---|---|---|---|---|
| | Moyenne | Ecart-type | Moyenne | Ecart-type |
| Total AGT (µg/mg poids sec, PS) | 48,9 | 8,6 | 40,5 | 7,0 |
| % de lipides neutres | 52,4 | 9,0 | 46,8 | 9,2 |
| Lipides polaires (µg/mg PS) | 20,9 | 1,6 | 20,0 | 2,0 |
| Lipides neutres (µg/mg PS) | 28,1 | 9,3 | 20,5 | 6,0 |

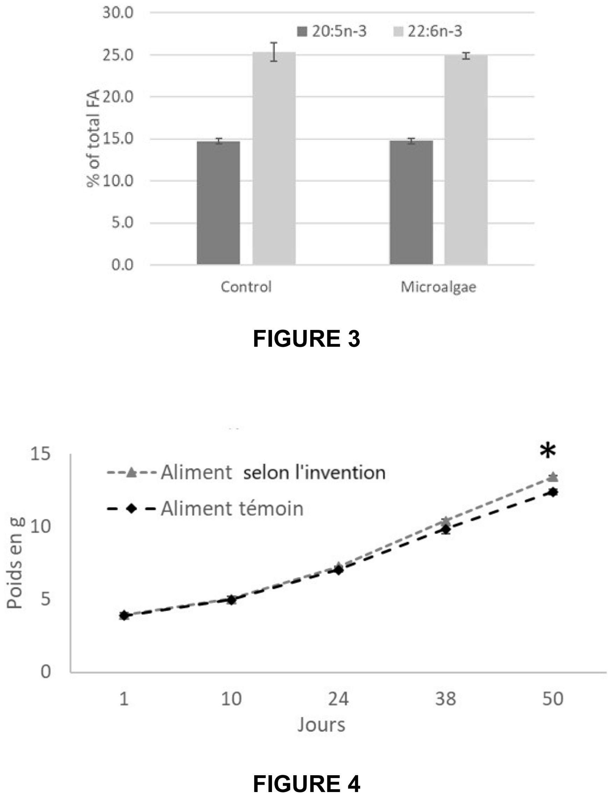

Il n'y a pas de différence significative entre les régimes pour les pourcentages d'EPA et de DHA dans les lipides neutres (voir Figure 2). Dans les lipides polaires, le pourcentage d'EPA est légèrement mais significativement plus élevé avec l'aliment selon l'invention par rapport à l'aliment témoin (voir Figure 3). Le pourcentage de DHA dans les lipides polaires varie entre 24 et 25% pour les deux régimes.

### 2. Influence des conditions nutritionnelles sur la cinétique de croissance des juvéniles de bar

Afin de valider l'intérêt de l'incorporation d'une biomasse selon l'invention dans l'aliment aquacole, des échantillonnages importants ont été réalisés tout au long de l'étude (voir tableau 9) et le poids moyen des juvéniles a été évalué au cours du temps (voir tableau 10).

**Tableau 9 : Prélèvement/bac au cours de l'étude (3 bacs / condition) avec initialement 2000 poissons/bac.**

| Contrôle de pesée N° | Durée de l'essai en jours | Effectif échantillonné par bac | Etapes du conditionnement nutritionnel |
|---|---|---|---|
| 1 | 1 | 200 | Début |
| 2 | 10 | 200 | |
| 3 | 24 | 200 | |
| 4 | 38 | 200 | Effectif par bac réduit à 500 par bac |
| 5 | 50 | 200 | Fin |

**Tableau 10 : Evolution des poids moyens des juvéniles de bar (g/poisson) par modalité au cours de l'essai**

| Jours | Aliment selon l'invention | | Aliment témoin | |
|---|---|---|---|---|
| | Moyenne | Ecart-type | Moyenne | Ecart-type |
| 1 | 3,96 | 0,13 | 3,90 | 0,17 |
| 10 | 5,05 | 0,15 | 4,98 | 0,26 |
| 24 | 7,25 | 0,04 | 7,05 | 0,14 |
| 38 | 10,42 | 0,08 | 9,85 | 0,35 |
| 50 | 13,44 | 0,08 | 12,42 | 0,19 |

Les courbes de croissance des juvéniles de bar sont présentées sur la Figure 4. L'allure générale des courbes de croissance est régulière et représentative d'une bonne croissance pour des lots de juvéniles de bars. Sur la base des moyennes observées, la modalité « aliment selon l'invention » reste assez proche de la modalité témoin jusqu'au contrôle de pesée N°3 (24 jours) puis présente des poids moyens supérieurs aux deux derniers échantillonnages avec une accentuation statistiquement significative (représentée par le symbole * sur la Figure 4) de cette différence de croissance à la pesée N°5 (50^{ème} jour).

Le gain de poids calculé à partir de la moyenne par bac est d'environ 8% pour les juvéniles de bar ayant consommé l'aliment contenant la biomasse d'A. *mangrovei* produite par le procédé selon l'invention en comparaison avec un aliment classique.

### 3. Conclusion

Les microorganismes A. *mangrovei* peuvent ainsi être cultivés dans un milieu nonaxénique composé majoritairement de coproduits de l'industrie laitière. Cette biomasse riche en DHA présente lorsqu'elle est incorporée à hauteur de 15% dans un aliment aquacole un double intérêt. Le premier est qu'elle permet de réduire de près de 10% l'utilisation de produits issus de la pêche minotière et de près de 5% l'utilisation de céréales à faible digestibilité pour sa formulation en comparaison avec un aliment « classique » tout en permettant de conserver une composition biochimique du poisson équivalente :
- teneur équivalente en acides gras essentiels,
- teneur en Oméga 3 similaire mais de façon intéressante nutritionnellement en plus grande proportion sous forme de lipides polaires.

Mais plus encore, en termes de croissance des juvéniles de bar, l'aliment contenant la biomasse selon l'invention permet un gain significatif de poids de près de 8% toujours en comparaison avec les poissons ayant consommé un aliment « classique ».

### EXEMPLE 4 : Influence des métaux traces et des vitamines dans le milieu de culture utilisé dans l'exemple 1 sur la production de biomasse de A. mangrovei

Les inventeurs ont étudié l'effet des métaux traces et des vitamines présents dans le milieu de culture utilisé dans l'exemple 1 sur la production de biomasse de A. *mangrovei.*

### Matériel et méthodes

12 réacteurs de 500 mL, divisés en 3 groupes, ont été utilisés :
- Groupe 1 : 3 réacteurs utilisant le milieu de culture de l'exemple 1 comprenant les solutions de vitamines et de métaux traces ;
- Groupe 2 : 3 réacteurs utilisant le milieu de culture de l'exemple 1 sans les solutions de métaux traces (*comprenant donc uniquement la saumure, le substrat carboné et les vitamines*) ;
- Groupe 3 : 3 réacteurs utilisant le milieu de culture de l'exemple 1 dans les solutions de métaux de traces et de vitamines *(comprenant donc uniquement la saumure et le substrat carboné*).

Trois séries d'expériences ont été réalisées.

Les inventeurs ont réalisé un comptage de l'inoculum de A. *mangrovei* (cultivé sur milieu YEP) puis ils ont incorporé 100 mL de cet inoculum dans une bouteille de 1500 mL du milieu de chacune des conditions. Ce volume a ensuite été réparti dans 3 réacteurs pour chaque groupe.

Les inventeurs ont ainsi calculé la concentration initiale théorique (C0 théorique) de A. *mangrovei,* qui correspond à la concentration calculée par rapport au volume d'inoculum introduit dans les bouteilles de 1500 mL.

Au terme de la culture, les inventeurs ont mesuré la concentration cellulaire dans les réacteurs.

Ils ont ainsi pu calculer la croissance cellulaire de A. *mangrovei* par rapport au C₀ théorique.

### Résultats

Les résultats obtenus sont présentés dans le tableau 11 ci-dessous.

**Tableau 11 : Comparaison de la croissance cellulaire de A. mangrovei dans différentes conditions de culture**

| **Groupe** | **N° réacteur** | **N° exp.** | **durée culture (h)** | **Croissance (%)** | **Moyenne** |
|---|---|---|---|---|---|
| | 1 | 1 | 47,75 | 137% | |
| | 1 | 2 | 43,25 | 71% | |
| | 1 | 3 | 68,25 | 255% | |
| **Groupe 1 (saumure + substrat carboné + vitamines + métaux traces** | 2 | 1 | 47,75 | 80% | **129%** |
| | 2 | 2 | 43,25 | 56% | |
| | 2 | 3 | 68,25 | 48% | |
| | 3 | 1 | 47,75 | 220% | |
| | 3 | 2 | 43,25 | 195% | |
| | 3 | 3 | 68,25 | 100% | |
| | 4 | 1 | 47,75 | -90% | |
| | 4 | 2 | 43,25 | -67% | |
| | 4 | 3 | 68,25 | -95% | |
| **Groupe 2 (saumure + substrat carboné + vitamines)** | 5 | 1 | 47,75 | -84% | **-74%** |
| | 5 | 2 | 43,25 | -26% | |
| | 5 | 3 | 68,25 | -77% | |
| | 6 | 1 | 47,75 | -85% | |
| | 6 | 2 | 43,25 | -74% | |
| | 6 | 3 | 68,25 | -69% | |
| **Groupe 3 (saumure + substrat carboné)** | 7 | 1 | 47,75 | -81% | **-64%** |
| | 7 | 2 | 43,25 | -51% | |
| | 7 | 3 | 68,25 | -95% | |
| | 8 | 1 | 47,75 | -62% | |
| | 8 | 2 | 43,25 | -13% | |
| | 8 | 3 | 68,25 | -77% | |
| | 9 | 1 | 47,75 | -49% | |
| | 9 | 2 | 43,25 | -64% | |
| | 9 | 3 | 68,25 | -86% | |

Les résultats montrent sans équivoque que la présence de métaux traces, et dans une moindre mesure de vitamines, dans le milieu de culture à base de saumure et de substrat carboné est indispensable pour la croissance des microorganismes de A. *mangrovei.* En l'absence des métaux traces et, dans une moindre mesure, des vitamines, les cellules meurent (une décroissance dans les réacteurs 7, 8 et 9 et ce, quelle que soit l'expérience).

### REFERENCES

1. Moomaw et al. (2017) Cutting out the Middle Fish: Marine Microalgae as the Next Sustainable Omega-3 Fatty Acids and Protein Source. Industrial Biotechnology 13 :243-243.
2. Tibaldi et al. (2006) Effects of the partial substitution of dietary fish meal by differently processed soybean meals on growth performance, nutrient digestibility and activity of intestinal brush border enzymes in the European sea bass (Dicentrarchus labrax) Aquaculture 261 :182-193.
3. Mundheim et al. (2004) Growth, feed efficiency and digestibility in salmon (Salmo Salar L.) fed different dietary proportions of vegetable protein sources in combination with two fish meal qualities. Aquaculture 236 :315-331.
4. Opstvedt et al. (2003) Efficiency of feed utilization in Atlantic salmon (Salmo salar L.) fed diets with increasing substitution of fish meal with vegetable proteins. Aquaculture 221 :365-379.
5. Bu et al. (2018) An Evaluation of Replacing Fishmeal with Rapeseed Meal in the Diet of Pseudobagrus ussuriensis: Growth, Feed Utilization, Nonspecific Immunity, and Growth-related Gene Expression. Journal of the World Aquaculture Society 49:1068-1080.
6. AFSSA Saisine n° 2008-SA-0316. Avis de l'Agence française de sécurité sanitaire des aliments relatif à la demande d'autorisation de mise sur le marché d'un nouvel aliment ou d'un ingrédient alimentaire: extension d'emploi de l'huile riche en DHA issue de la micro-algue Schizochytrium sp. (2008).
7. Soudant et al. (2022). Evaluation of Aurantiochytrium mangrovei Biomass Grown on Digestate as a Sustainable Feed Ingredient of Sea Bass, Dicentrarchus labrax, Juveniles and Larvae. Sustainability 14:14573.
8. Choubert et al. (1982) Digestibility in fish: Improved device for the automatic collection of feces. Aquaculture 29:185-189.

## Revendications

1. Procédé de préparation d'une biomasse de microorganismes du genre *Schizochytrium* ou *Aurantiochytrium* comprenant les étapes de :
a) ensemencement, dans un bioréacteur, d'un milieu de culture comprenant une saumure, de préférence une saumure 6% d'extrait sec, et de l'azote, de préférence à une concentration comprise entre 5 et 15 g/L de milieu de culture, avec un inoculum de microorganismes du genre *Schizochytrium* ou *Aurantiochytrium,* ladite saumure étant un coproduit du traitement de déminéralisation du petit-lait, et le milieu de culture comprenant en outre au moins un substrat carboné et des métaux traces,
b) culture des microorganismes dans ledit milieu de culture dans des conditions, notamment de température, pH, agitation et/ou apport en oxygène, appropriées pour la croissance desdits microorganismes, de préférence en conditions non-axéniques, et
c) collecte de la biomasse obtenue à l'issue de l'étape b).

2. Procédé selon la revendication 1, dans lequel les microorganismes du genre *Schizochytrium* ou *Aurantiochytrium* sont des microorganismes de l'espèce *Aurantiochytrium mangrovei.*

3. Procédé selon la revendication 1 ou 2, dans lequel l'azote est de l'azote ammoniacal.

4. Procédé selon l'une quelconque des revendications 1 à 3, dans lequel le milieu de culture comprend en outre au moins une vitamine.

5. Procédé selon l'une quelconque des revendications 1 à 4, dans lequel les microorganismes sont cultivés à l'étape b) à une température comprise entre 27°C et 30°C et/ou à un pH compris entre 6 et 7.

6. Procédé selon l'une quelconque des revendications 1 à 5, dans lequel la biomasse de microorganismes est collectée à l'étape c) lorsque la concentration en biomasse dans le milieu de culture a atteint au moins 10 g/L et/ou après 42h de culture.

7. Procédé selon l'une quelconque des revendications 1 à 6, comprenant en outre, après l'étape c), une étape de filtration, et optionnellement une étape de congélation et/ou de lyophilisation de ladite biomasse.

8. Biomasse de microorganismes du genre *Schizochytrium* ou *Aurantiochytrium* susceptible d'être obtenue par le procédé selon l'une quelconque des revendications 1 à 7.

9. Biomasse de microorganismes du genre *Schizochytrium* ou Aurantiochytrium **caractérisée en ce qu'**elle présente le profil lipidique suivant :
- entre 55 et 65% d'acides gras totaux sous forme de lipides polaires, et
- au moins une des caractéristiques suivantes :
• entre 18 et 25 µg d'acides gras totaux / mg de poids de sec de biomasse,
• entre 35 et 40% d'acide docosahexaénoïque (DHA) dans les lipides neutres,
• un ratio DHA / acide docosapentaénoïque (DPA) dans les lipides neutres compris entre 4 et 4,5,
• entre 45 et 55% de DHA dans les lipides polaires, et
• un ratio DHA / DPA dans les lipides polaires compris entre 3 et 4.

10. Biomasse selon la revendication 8, **caractérisée par** le profil lipidique défini dans la revendication 9.

11. Utilisation de la biomasse de microorganismes du genre *Schizochytrium* ou *Aurantiochytrium* selon la revendication 8 ou 9 comme ingrédient dans un aliment pour animaux d'élevage, notamment un aliment aquacole, en particulier dans un aliment pour bar d'élevage.

12. Aliment pour animaux d'élevage, notamment aquacole, en particulier pour bar d'élevage, comprenant entre 15% et 30% en poids de biomasse de microorganismes du genre *Schizochytrium* ou *Aurantiochytrium* selon l'une quelconque des revendications 8 à 10.

13. Aliment selon la revendication 12, comprenant moins de 50 % en poids d'ingrédient issu de la pêche minotière et/ou comprenant moins de 10% en poids de céréales à faible digestibilité.

14. Méthode d'élevage de poissons, en particulier de bars, plus particulièrement de juvéniles de bars, comprenant la fourniture, notamment pendant 5 à 10 semaines, de préférence tous les jours, de la biomasse de microorganismes du genre *Schizochytrium* ou *Aurantiochytrium* selon l'une quelconque des revendications 8 à 10 auxdits poissons, de préférence sous la forme de l'aliment selon la revendication 12 ou 13, de préférence à une dose moyenne journalière de 2% à 3% du poids vif.
